(19) Europäisches Patentamt
European Patent Office
Office européen des brevets

(11) Veröffentlichungsnummer: **0 443 370 B1**

# (12) EUROPÄISCHE PATENTSCHRIFT

(45) Veröffentlichungstag der Patentschrift: **24.08.94**

(51) Int. Cl.5: **C07C 69/92**, C07C 271/28, C07C 265/12, C07C 67/14, C07C 269/02, C08F 120/06, C08F 120/36, A61K 6/083

(21) Anmeldenummer: **91101639.2**

(22) Anmeldetag: **07.02.91**

## (54) Dentalwerkstoffe.

(30) Priorität: **20.02.90 DE 4005231**

(43) Veröffentlichungstag der Anmeldung:
**28.08.91 Patentblatt 91/35**

(45) Bekanntmachung des Hinweises auf die Patenterteilung:
**24.08.94 Patentblatt 94/34**

(84) Benannte Vertragsstaaten:
**AT BE CH DE DK ES FR GB GR IT LI LU NL SE**

(56) Entgegenhaltungen:
**EP-A- 0 201 778**
**EP-A- 0 343 441**
**US-A- 4 356 296**

(73) Patentinhaber: **BAYER AG**
**D-51368 Leverkusen (DE)**

(72) Erfinder: **Müller, Michael, Dr.**
**Richard-Zanders-Strasse 34**
**W-5060 Bergisch Gladbach 2 (DE)**
Erfinder: **Podszun, Wolfgang, Dr.**
**Roggendorfstrasse 55**
**W-5000 Köln 80 (DE)**
Erfinder: **Winkel, Jens, Dr.**
**Letterhausstrasse 1**
**W-5000 Köln 71 (DE)**
Erfinder: **Negele, Michael, Dr.**
**Wolfskaul 6**
**W-5000 Köln 80 (DE)**

**EP 0 443 370 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Acrylsäure- und Methacrylsäureester substituierter 1,2-Bis(phenoxy)-3,3,4,4,5,5-hexafluor-1-cyclopentene, ihre Herstellung und ihre Verwendung als Monomere für die Anwendung im Dentalbereich.

In konventionellen Zahnfüllmassen, Zahnlacken und speziellen Klebstoffen für dentale Anwendungen besteht die organische Matrix u.a. aus 2,2-Bis-[4'(3''-methacryloyl-2''-hydroxypropoxy)phenyl]-propan (Bis-GMA). Die daraus durch Polymerisation hergestellten Kunststoffe sind relativ stark hydrophil und erfahren im Mund des Patienten eine Degradation durch kombinierte chemische und physikalische Beanspruchung. Aufgrund ihre Hydrophilie nehmen diese Kunststoffe im feuchten Mundmilieu Wasser und in ihm befindliche Chemikalien und Bakterien auf, die z.B. beim Verdauungsprozeß im Mund entstehen. Dies führt über einen längeren Zeitraum zu einer Abnahme der Härte und Verschleißfestigkeit dieser Materialien und fördert zudem den Plaquebefall und damit die Bildung von Sekundärkaries.

Zur Hydrophobierung von Dentalmaterialien ist bereits der Einsatz fluorhaltiger Verbindungen bekannt. Fluorhaltige Phenylcarbinolacrylate wie 1,1,1,3,3,3-Hexafluor-2-phenyl-2-acryloyloxy-propan sind aus Org. Coat. Plast. Chem. 42 (1980) 204 bekannt. Ähnlich aufgebaute (Meth)acrylsäureester, wie 1,3-Bis-(2-(meth)-acryloyloxy-1,1,1,3,3,3-hexafluoropropyl-2)-5-perfluoralkyl-benzol und ihre Verwendung auf dem Dentalgebiet werden in der US 4 356 296 beschrieben. Durch die Trifluormethylgruppen werden die Carbinole acidifiziert und die daraus hergestellten Carbinolester zeichnen sich durch eine verminderte Hydrolysenbeständigkeit aus. Dadurch ist ihre Verwendbarkeit als Dentalmonomere eingeschränkt.

Weiterhin ist die Verwendung von 1,1,5-Trihydro-octafluoro-pentyl-methacrylatin Zahnfüllmassen in J. Dent. Res. 58 (1979) 1181 beschrieben (US 4 292 029). Monomere dieses Typs liefern Dentalmaterialien mit niedrigem mechanischen Eigenschaftsniveau und sind in ihrem Verarbeitungsverhalten und aus toxikologischen Erwägungen aufgrund ihres hohen Dampfdruckes kritisch zu beurteilen. Trifluormethylgruppen enthaltende (Meth)acrylate nach EP 295 639 entstehen durch sukzessive Umsetzung von Hexafluor-2-propanol-Einheiten mit dem carzinogenen Epichlorhydrin, Base und Methacrylsäure, wobei das entstehende Produkt stark hydrophile Hydroxypropylgruppen enthält. In Polym. Mater. Sci. Eng. 59 (1988) 388 werden polyfluorierte Methacrylate ("PFMA", US 4 616 073) und Urethanmethacrylate ("PFUMA") beschrieben, die sich in einer polymeranalogen Reaktion aus einem polyfluorierten Polyol bilden. Die Vollständigkeit der Umsetzung der OH-Funktionen des Polyols muß dabei ebenso kritisch beurteilt werden, wie die Verfügbarkeit eingesetzter Chemikalien wie dem 2-Isocyanatoethylmethacrylat.

(Meth)acrylsäureester mit einem zentralen 1,2-Diphenyl-1,1,2,2-tetrafluorethan-Baustein sind zudem aus der DE-OS 35 16 256 und DE-OS 35 16 257 bekannt.

Es wurden nun neue (Meth)acrylsäureester substituierter 1,2-Bis-(phenoxy)-3,3,4,4,5,5-hexafluor-1-cyclopentene der Formel (I)

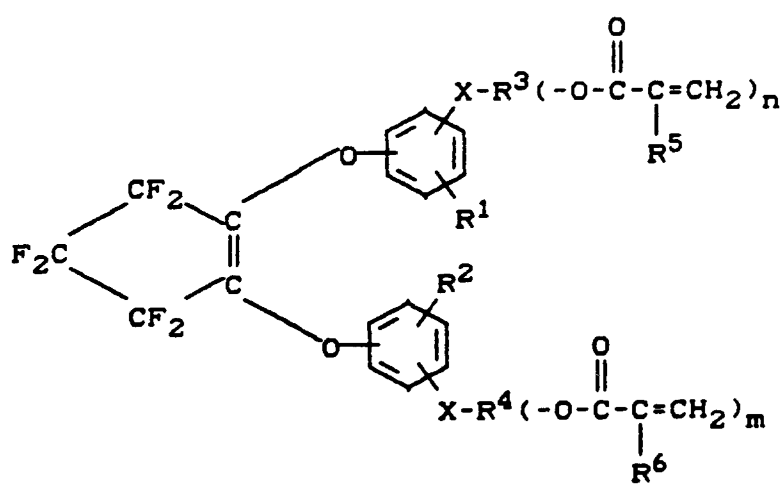

(I)

gefunden, in der jeweils unabhängig voneinander

$R^1$, $R^2$ $C_1$- bis $C_4$-Alkyl, $C_1$- bis $C_4$-Halogenalkyl oder Wasserstoff bedeuten,

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können,

bedeuten,

$R^5$, $R^6$ Wasserstoff oder Methyl bedeuten und

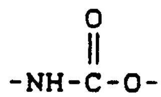 oder 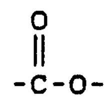

X für steht und

n und m ganze Zahlen von 1 bis 5 bedeuten.

Im Rahmen der vorliegenden Erfindung können die Substituenten im allgemeinen folgende Bedeutung haben;

$C_1$- bis $C_4$-Alkyl kann einen geradkettigen oder verzweigten Kohlenwasserstoffrest mit 1 bis 4 Kohlenstoffatomen bedeuten. Beispielsweise seien die folgenden Alkylreste genannt: Methyl, Ethyl, Propyl, Isopropyl, Butyl und Isobutyl. Bevorzugt ist der Methylrest. Als besonders bevorzugt für die Reste $R^1$ und $R^2$ gilt Wasserstoff. Bei $C_1$- bis $C_4$-Halogenalkylresten kann es sich um Reste mit einem oder mehreren, gleichen oder verschiedenen Halogenatomen handeln. Als Halogenatome kommen Fluor, Chlor, Brom und/oder Iod in Frage, bevorzugt sind Fluor, Chlor und/oder Brom, insbesondere Fluor und/oder Chlor. Das bzw. die Halogenatome können innenständig oder endständig angeordnet sein. Eine besonders bevorzugte Gruppe von $C_1$- bis $C_4$-Halogenalkylresten sind die Perfluoralkylreste; ganz besonders bevorzugt ist die Trifluormethylgruppe. Im Rahmen der Substituenten $R^3$ und $R^4$ können die (n + 1)- bzw. (m + 1)-wertigen Kohlenwasserstoffreste gerad- oder verzweigtkettig sein und 2 bis 15, bevorzugt 2 bis 10 und besonders bevorzugt 2 bis 5, Kohlenstoffatome enthalten. Diese Kohlenwasserstoffreste für $R^3$ und $R^4$ können gegebenenfalls auch 1 bis 3 Sauerstoffbrücken, bevorzugt 1 oder 2 Sauerstoffbrücken, enthalten.

Die Wertigkeiten der Substituenten werden durch n und m bestimmt. n und m stehen unabhängig voneinander für ganze Zahlen von 1 bis 5, bevorzugt 1 und 2.

Beispielsweise seien die folgenden Reste für die Substituenten $R^3$ und $R^4$ genannt:

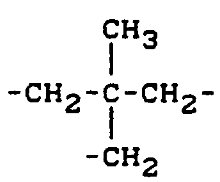 , 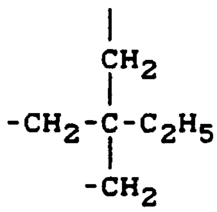 , 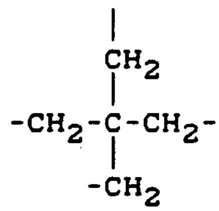 ,

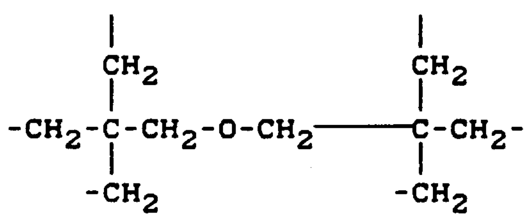 , 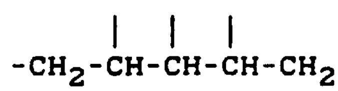 ,

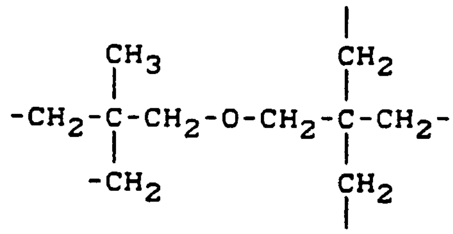 , 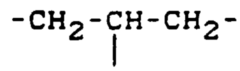 ,

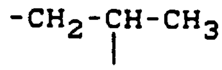 ,

$-CH_2CH_2-$ .

Die Reste $R^5$ und $R^6$ stehen für Wasserstoff oder Methyl. Dies findet im Rahmen vorliegender Erfindung Ausdruck in der Bezeichnung (Meth)acrylsäureester, womit Ester der Acryl- oder Methacrylsäure gemeint sind, Bevorzugt für die Reste $R^5$ und $R^6$ wird Methyl.

Die neuen erfindungsgemäßen (Meth)acrylsäureester sind farblose, schwerflüchtige, niederviskose Öle und ergeben nach Polymerisation transparente Kunststoffe, Im Rahmen der vorliegenden Erfindung ist es ebenfalls bevorzugt, Mischungen verschiedener erfindungsgemäßer (Meth)acrylsäureester einzusetzen, Sie lassen sich besonders gut in Abdichtungsmitteln, Klebstoffen und vorzugsweise Dentalmaterialien, wie Zahnfüllmassen und Beschichtungsmitteln, verwenden. Die so erhaltenen Materialien zeichnen sich durch eine überraschend große Widerstandfähigkeit gegenüber physikalischer und chemischer Beanspruchung aus. In besonderem Maße sind die mechanischen Eigenschaften gegenüber üblichen, zu diesem Zweck eingesetzten Materialien, verbessert, Besonders hervorzuheben sind die günstigen Oberflächeneigenschaften und geringe Wasseraufnahme der mit den neuen (Meth)acrylsäureestern erhaltenen Polymerisate.

Bevorzugte (Meth)acrylsäureester substituierter 1,2-Bis-(phenoxy)-3,3,4,4,5,5-hexafluor-1-cyclopentene sind Verbindungen der Formel (I)

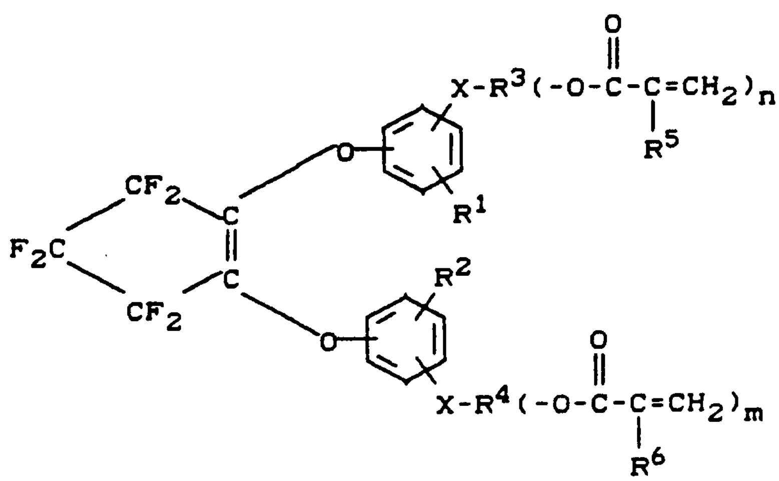

(I)

in der jeweils unabhängig voneinander

| | |
|---|---|
| $R^1$, $R^2$ | Wasserstoff oder Trifluormethyl bedeuten, |
| $R^3$, $R^4$ | (n + 1)- bzw. (m + 1)-wertige, geradkettige oder verzweigte Kohlenwasserstoffreste mit 2 bis 15 Kohlenstoffatomen bedeuten, |
| $R^5$, $R^6$ | Wasserstoff oder Methyl bedeuten und |
| X | für |

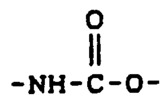 oder 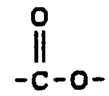

steht und

n und m 1 oder 2 bedeuten.

Beispielsweise seien die folgenden erfindungsgemäßen (Meth)acrylsäureester genannt, wobei für den 3,3,4,4,5,5-Hexafluor-1-cyclopenten-Ring das Symbol

" 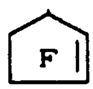 "

gewählt wird.

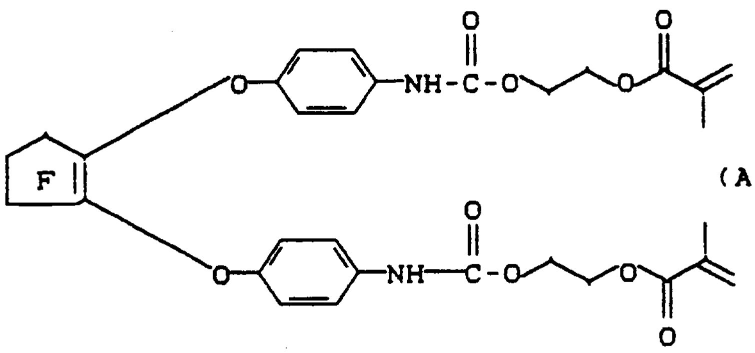

($A-HEMA_2$: s.Bsp.1)

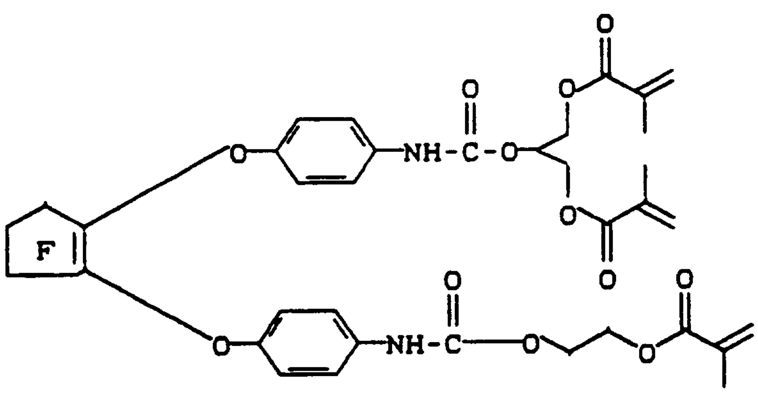

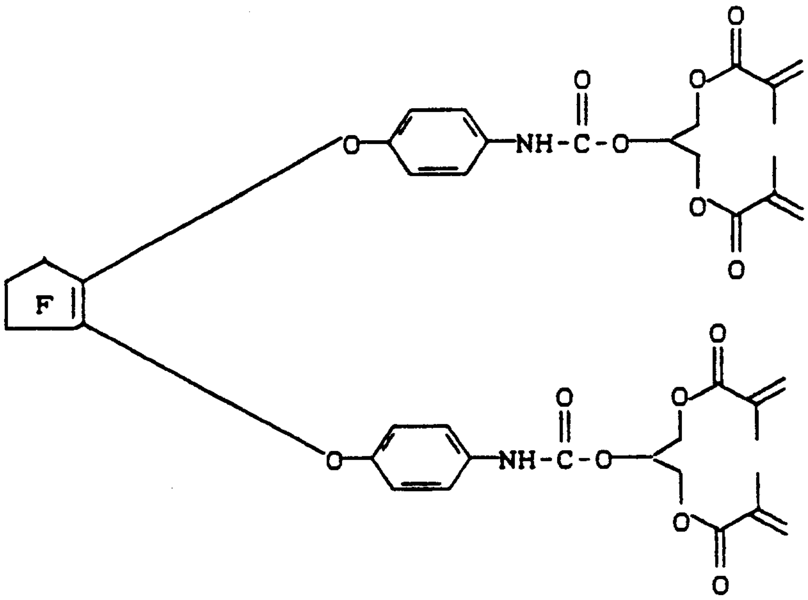

(A-GDMA$_2$: s.Bsp.2)

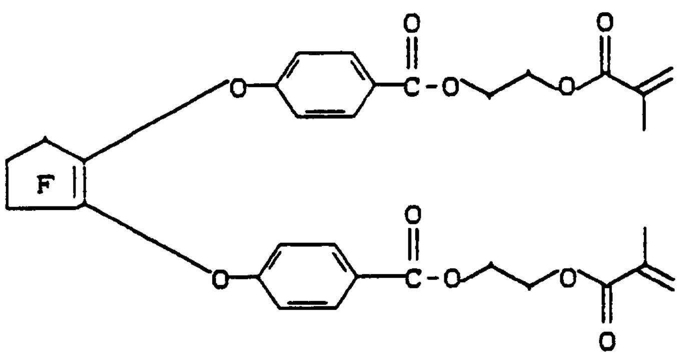

(B-HEMA$_2$: S.Bsp.3)

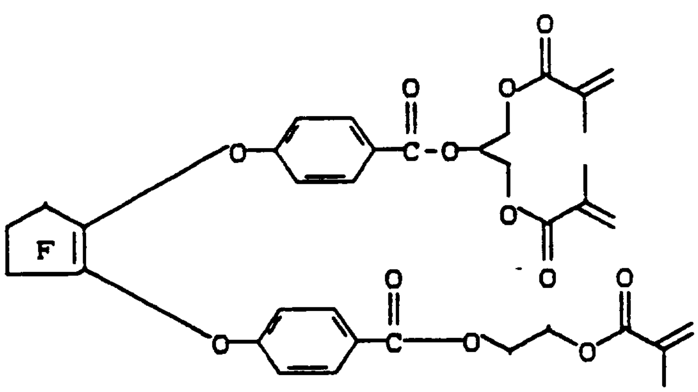

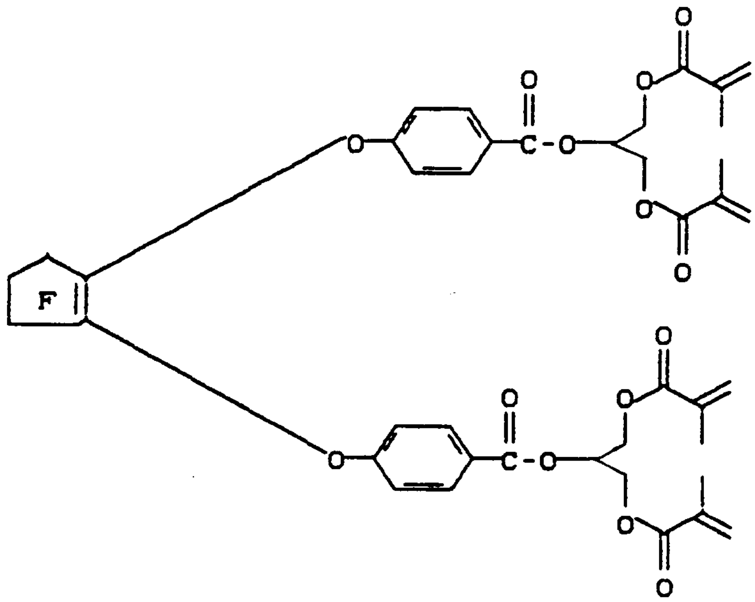

B-GDMA$_2$:
s.Bsp.4)

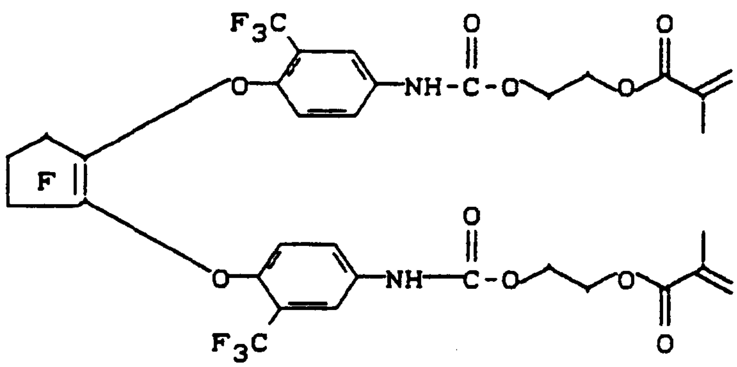

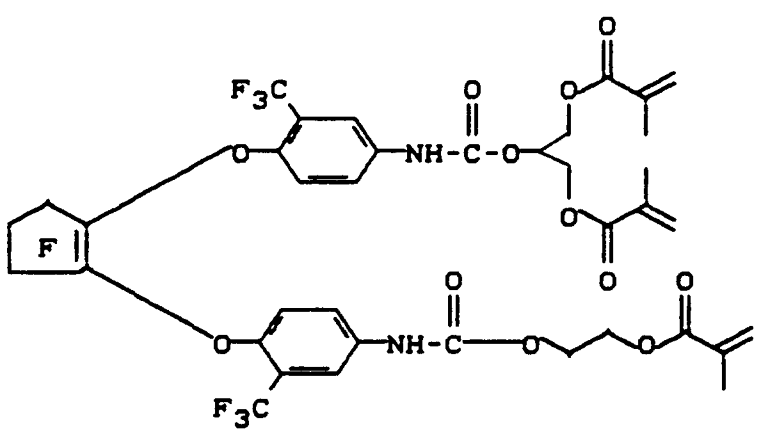

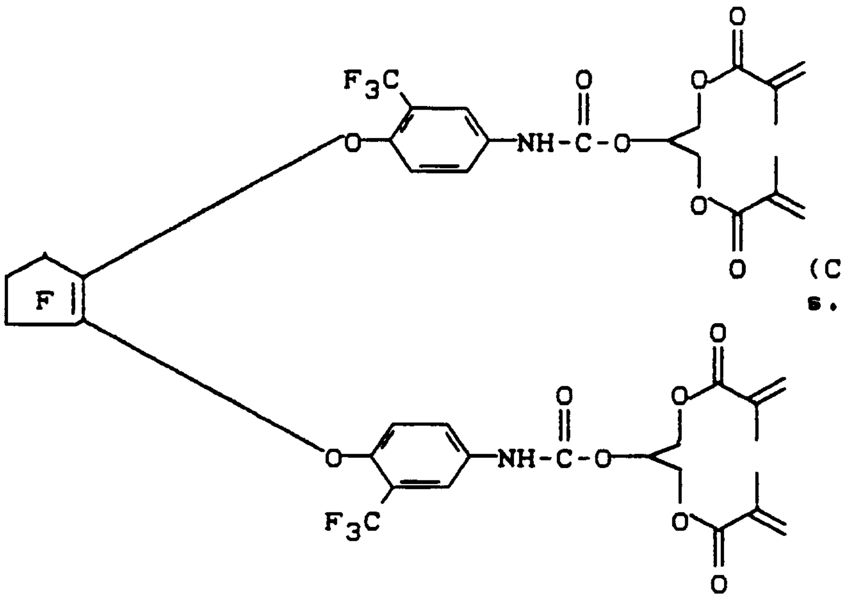

(C-GDMA$_2$: s. Bsp. 5)

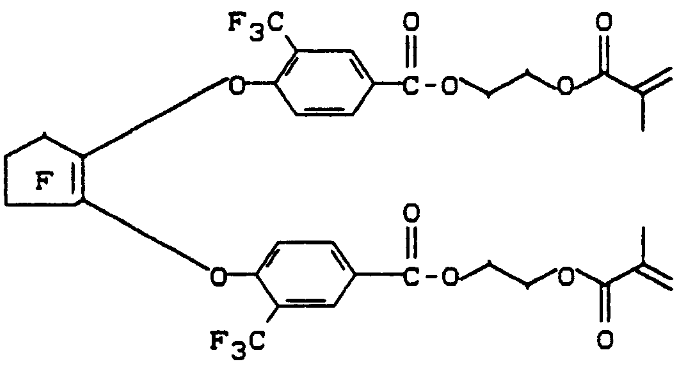

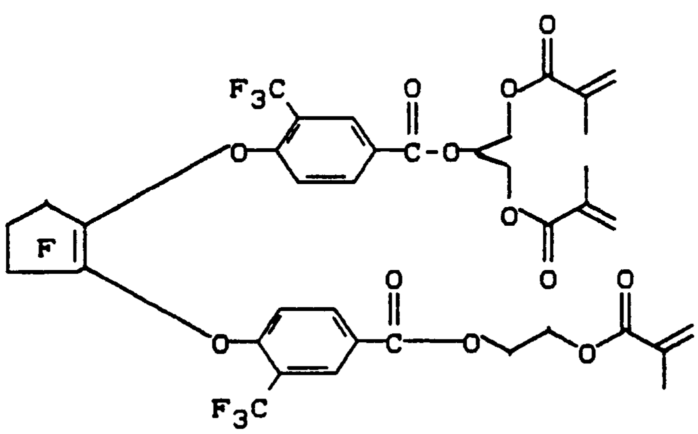

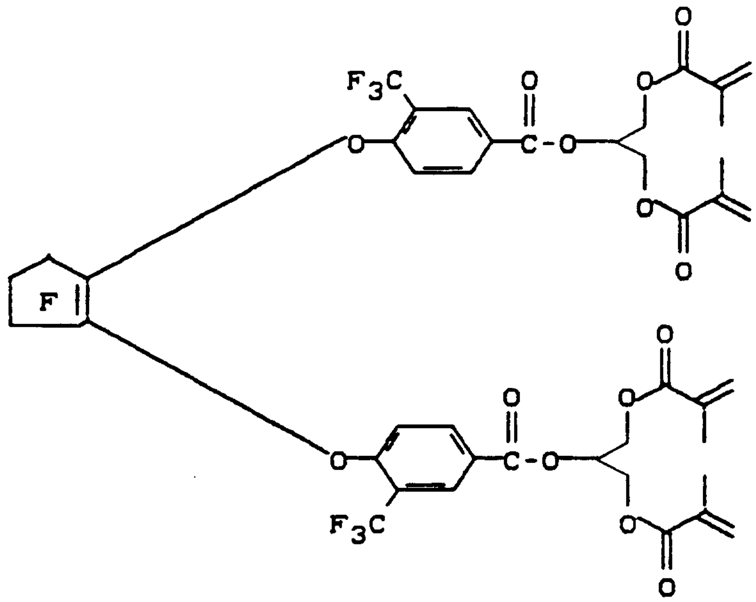

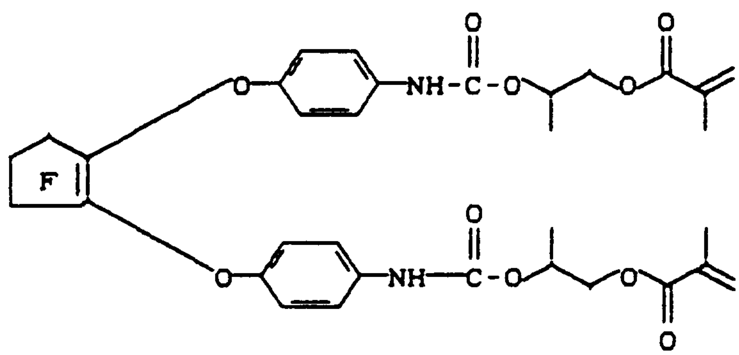

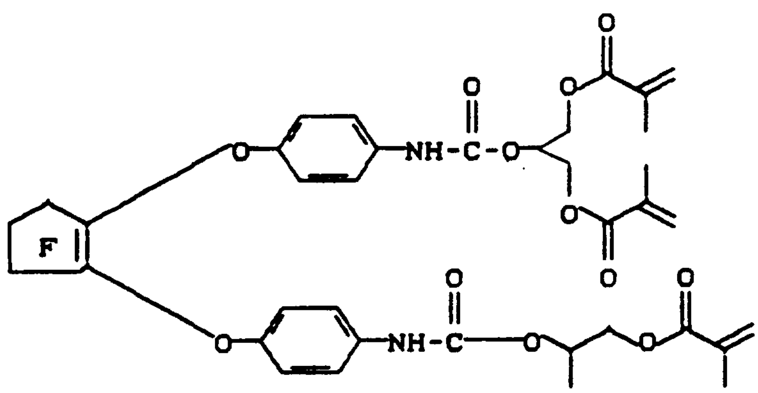

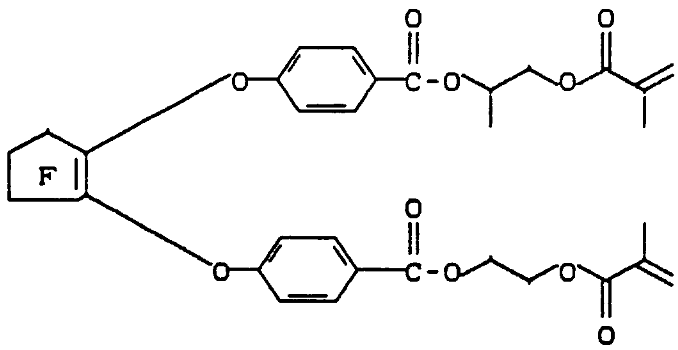

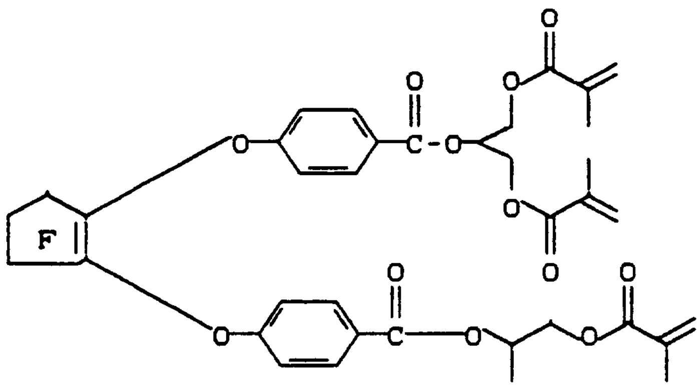

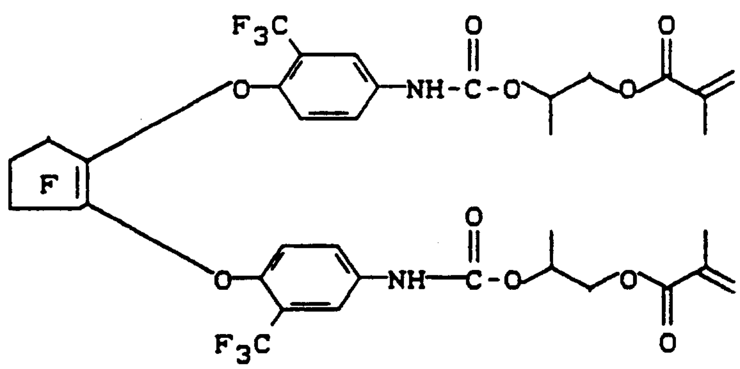

($C$-$HPMA_2$: s. Bsp. 5)

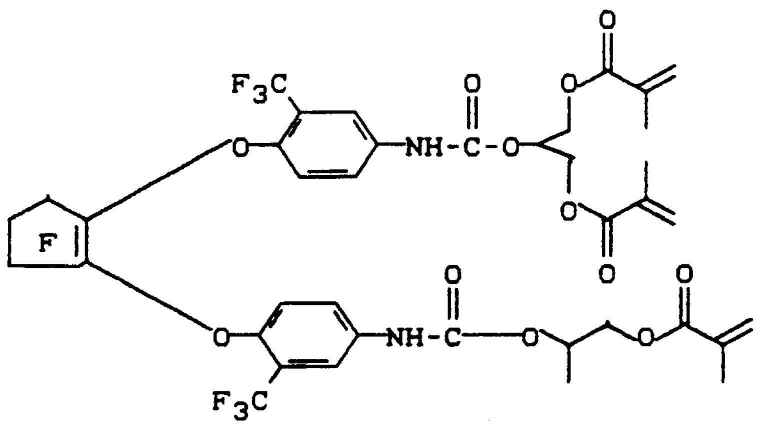

(C-GDMA-HPMA: s. Bsp. 5)

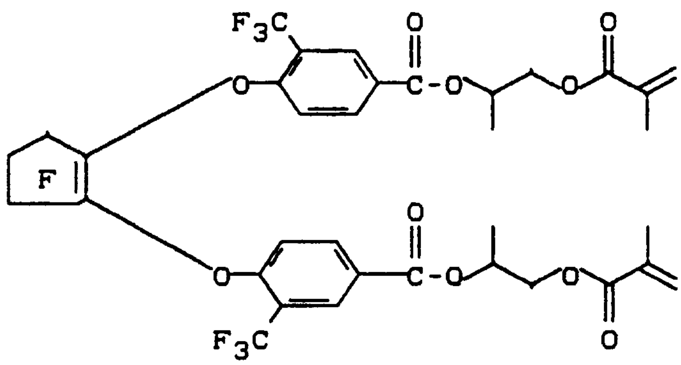

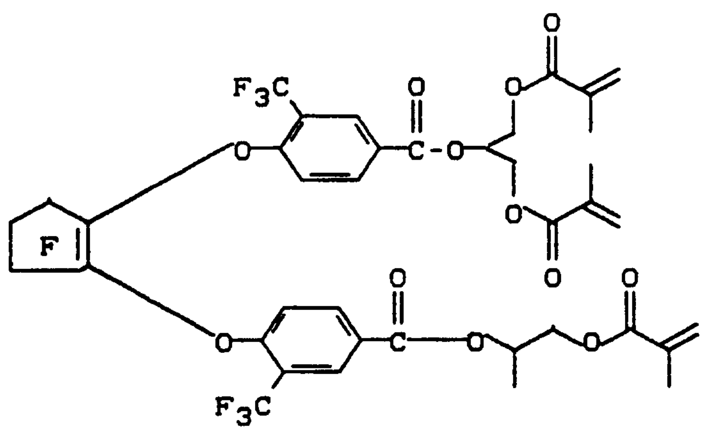

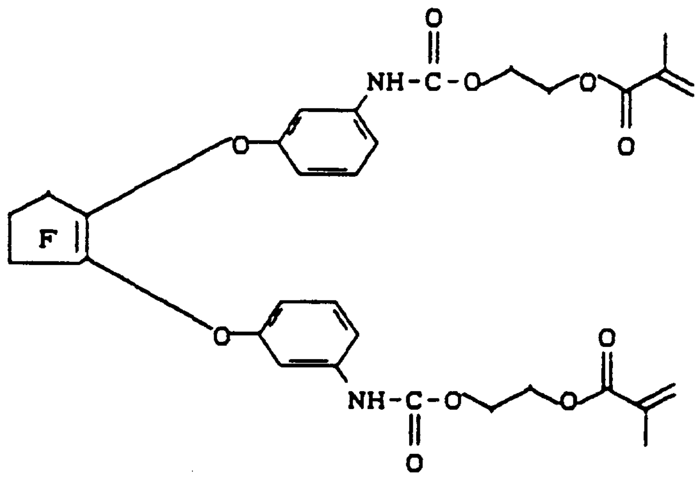

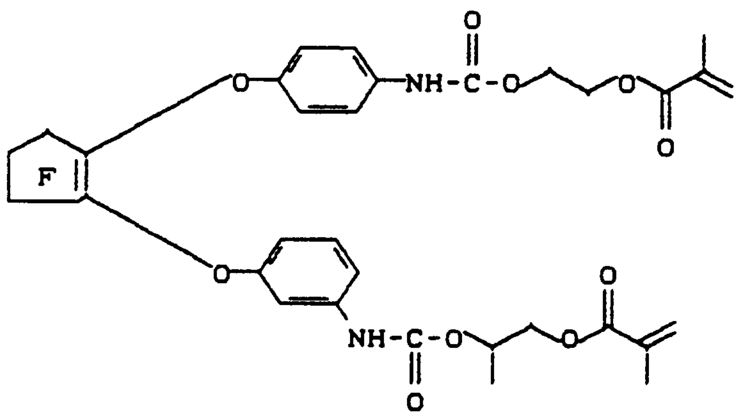

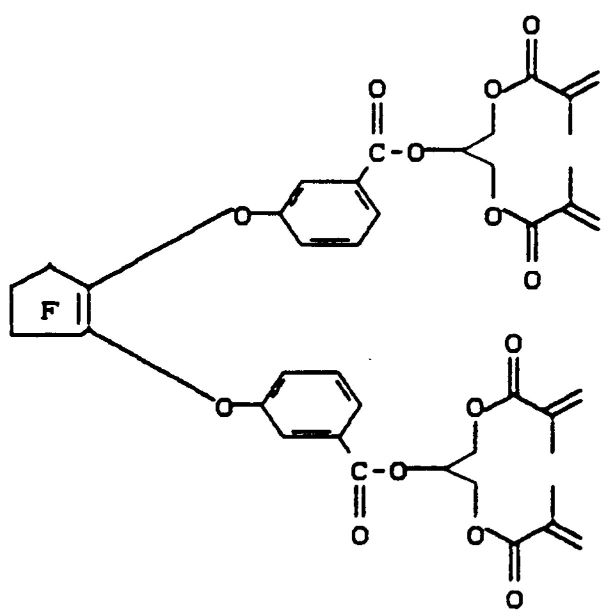

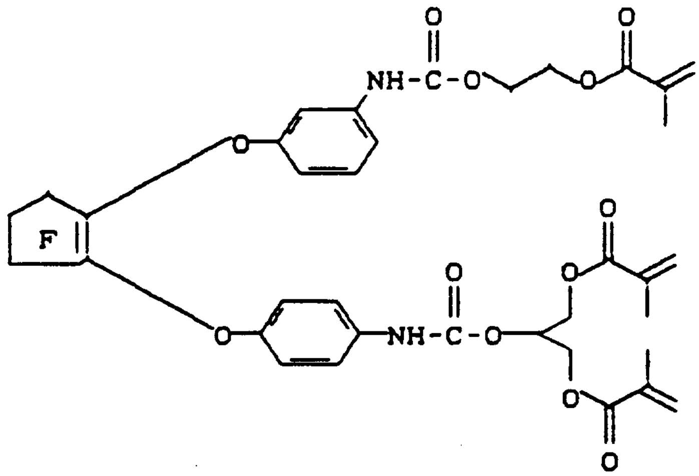

Es wurde weiterhin ein Verfahren zur Herstellung von (Meth)acrylsäureestern der Formel (I) gefunden, das dadurch gekennzeichnet ist, daß man substituierte 1,2-Bis-(phenoxy)-3,3,4,4,5,5-hexafluor-1-cyclopentene der Formel

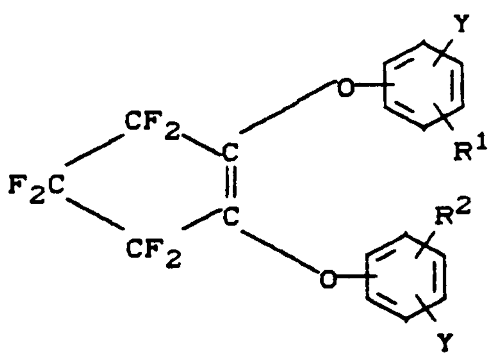

(III)

worin

$R^1$, $R^2$ unabhängig voneinander $C_1$- bis $C_4$-Alkyl, $C_1$- bis $C_4$-Hologenalkyl oder Wasserstoff bedeuten und

Y für Isocyanato (-NCO) oder Chlorocarbonyl (-COCl) steht,

mit mindestens einem OH-funktionellen (Meth)acrylsäure-Derivat der Formel

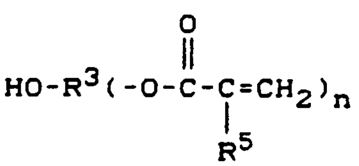

oder

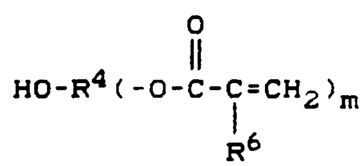

(IV) (V)

worin unabhängig voneinander

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten,

$R^5$, $R^6$ Wasserstoff oder Methyl bedeuten und

n und m ganze Zahlen von 1 bis 5 bedeuten

in einem inerten Lösungsmittel, im Temperaturbereich zwischen -40 und +100 °C, gegebenenfalls in Gegenwart eines Katalysators und/oder einer Base sowie gegebenenfalls in Gegenwart eines Polymerisationsinhibitors umsetzt.

Die Herstellung der für das erfindungsgemäße Verfahren benötigten substituierten 1,2-Bis(phenoxy)-3,3,4,4,5,5-hexafluor-1-cyclopentene der Formel (III) ist aus der DE-OS 3 817 626 bekannt. Als geeignete Verbindungen der Formel (III) seien genannt:

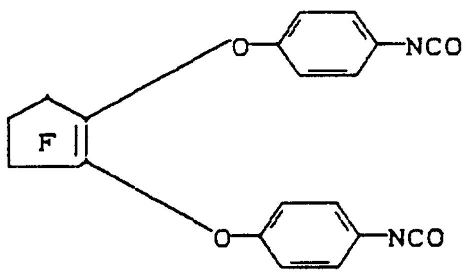

(A; s. Bsp. 1 und 2),

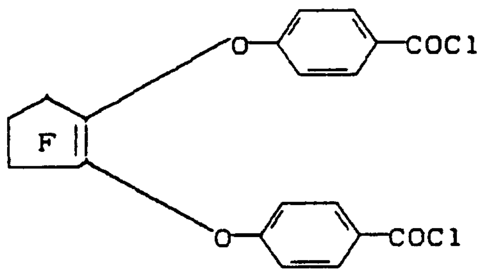

(B; s. Bsp. 3 und 4),

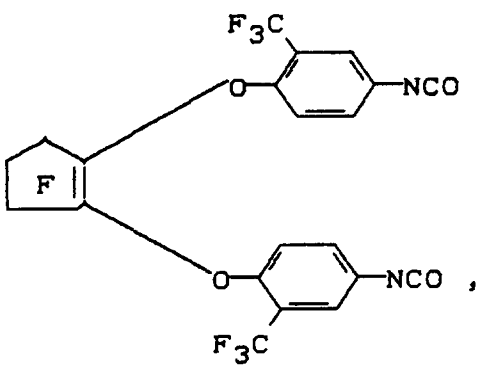

(C; s. Bsp. 5)

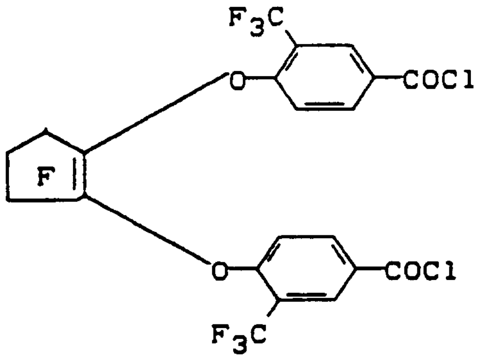
,

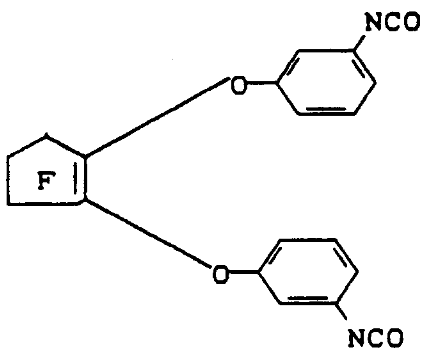
,

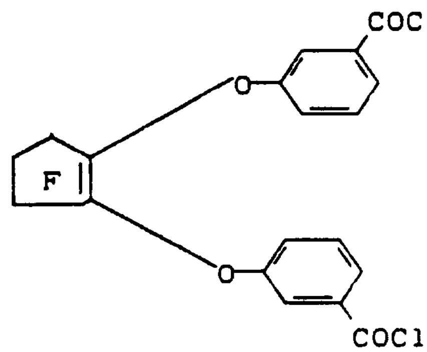
,

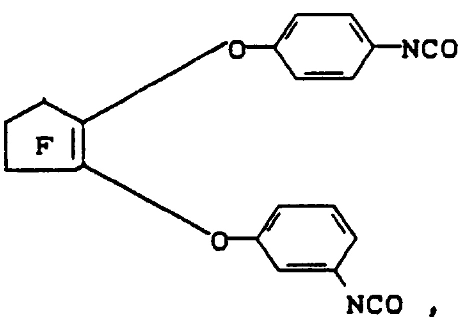
,

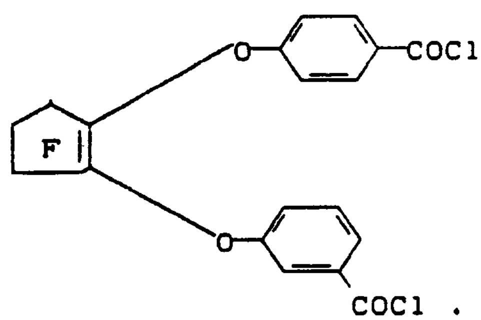
.

OH-funktionelle (Meth)acrylsäure-Derivate der Formeln (IV) und (V) sind handelsüblich oder können in bekannter Weise durch partielle Veresterung der entsprechenden Polyole

$HO-R^3(OH)_n$

oder

$HO-R^4(OH)_m$

hergestellt werden.

Als geeignete OH-funktionelle (Meth)acrylsäure-Derivate seien genannt:

Dodekandiolmono(meth)acrylat, Decandiolmono(meth)acrylat, Nonandiolmono(meth)acrylat, Octandiolmono(meth)acrylat, Heptandiolmono(meth)acrylat, Polyethylenglykolmono(meth)acrylate mit mehr als vier Ethoxyeinheiten, Polypropylenglykolmono(meth)acrylate mit mehr als vier Propoxyeinheiten, Pentaerythrittri(meth)acrylat und Dipentaerythritpenta(meth)acrylat.

Als bevorzugte OH-funktionelle (Meth)acrylsaure-Derivate der Formeln (IV) und (V) seien Hexandiolmono(meth)acrylat, Pentandiolmono(meth)acrylat, Butandiolmono(meth)acrylat,

Tetraethylenglykolmono(meth)acrylat, Tetrapropylenglykolmono(meth)acrylat und Trimethylolpropandi(meth)acrylat genannt.

Besonders bevorzugte OH-funktionelle (Meth)acrylsäure-Derivate sind 3-Hydroxypropyl(meth)acrylat, 2-Hydroxyethyl(meth)acrylat, Tri- und Diethylenglykolmono(meth)acrylat, Tri- und Dipropylenglykolmono(meth)acrylat und Glycerindi(meth)acrylat.

Existieren Isomere der genannten OH-funktionellen (Meth)acrylsäure-Derivate der Formeln (IV) und (V) bezüglich der Stellung der (Meth)acryloyl-Gruppierung bzw. bezüglich des zentralen Kohlenwasserstoffskeletts (-verzweigt bzw. unverzweigt-), so ist die Verwendung von Isomerengemischen bevorzugt.

Die Umsetzung der Diisocyanate und Säurechloride gemäß Formel (III) zu den erfindungsgemäßen (Meth)acrylsäureestern (I) erfolgt vorzugsweise unter Wasserausschluß in einem inerten Lösungsmittel. Beispiele für geeignete Lösungsmittel sind: Chloroform, Tetrahydrofuran, Dioxan, Methylenchlorid, Toluol, Acetonitril und Frigene. Bevorzugte Lösungsmittel sind Chloroform, Tetrahydrofuran, Frigen 113, Acetonitril und Methylenchlorid.

Insbesondere kann das erfindungsgemäße Verfahren auch in einem niedrig viskosen Comonomer als Lösungsmittel erfolgen, das selbst (Meth)acrylatgruppen enthält und deshalb nach der Reaktion nicht entfernt werden muß, da es mit den erfindungsgemäßen Verbindungen copolymerisierbar ist. Das erhaltene Monomergemisch ist so direkt zur Herstellung von Dentalmaterialien geeignet. Geeignete Reaktivverdünner sind Di(meth)acrylate von zweiwertigen Alkoholen wie Alkandiolen oder Ethylenglykolen und Propylenglykolen mit zwei bis zwölf Kohlenstoffatomen. Besonders geeignet sind Hexandioldimethacrylat und Triethylenglykoldimethacrylat.

Die Umsetzung wird im allgemeinen im Temperaturbereich von -40 bis +100 °C, vorzugsweise -35 bis +70 °C, durchgeführt.

Zur Beschleunigung der Umsetzung der Diisocyanate gemäß Formel (III) werden gegebenenfalls zinnhaltige Katalysatoren wie Dibutylzinndilaurat oder Zinn(II)octoat verwendet. Andere geeignete Katalysatoren sind Verbindungen mit tert. Aminogruppen und Titanverbindungen. Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden, eingesetzt.

Die Umsetzung der Säurechloride gemäß Formel (III) kann gegebenenfalls in Gegenwart von Basen erfolgen. Geeignete Basen sind Natriumhydroxid, Kaliumhydroxid, Natrium- oder Kaliumcarbonat, Natrium- oder Kaliumhydrogencarbonat sowie tertiäre Amine wie Triethylamin, Pyridin, N-Methylpiperidin, 1,5-Diazabicyclo[4.3.0]non-5-en oder 1,8-Diazabicyclo[5.4.0]undec-7-en.

Das erfindungsgemäße Verfahren wird im allgemeinen vorteilhaft in Gegenwart von 0,01 bis 0,2 Gew.-% eines Polymerisations-Inhibitors, bezogen auf die Gesamtmenge der Reaktanden, unter Normaldruck durchgeführt. Es ist aber auch möglich, das erfindungsgemäße Verfahren bei einem Unter- oder Überdruck durchzuführen.

Ein geeigneter Inhibitor ist zum Beispiel 2,6-Di-tert.-butyl-p-kresol. Geeignet ist auch Luft, die in das Reaktionsgemisch eingeleitet wird. Das erfindungsgemäße Verfahren kann beispielsweise wie folgt durchgeführt werden:

Die Reaktanden werden in dem Lösungsmittel gelöst und unter Rühren gegebenenfalls mit dem Katalysator und/oder der Base versetzt. Der zeitliche Verlauf der Umsetzung kann beispielsweise durch Messung der IR-Spektren verfolgt werden. Nach vollständiger Umsetzung der Isocyanat-bzw. Säurechloridgruppen werden die Reaktionsprodukte nach Filtration enthaltener Feststoffe durch Entfernen des Lösungsmittels isoliert. Eine vorherige Reinigung mit Hilfe von Adsorbentien, beispielsweise Aktivkohle, Bleicherde, Kieselgel oder Aluminiumoxid ist möglich.

Für die Anwendung als Monomere für polymere Zahnfüllmassen oder Beschichtungsmittel (Zahnlacke) im Dentalbereich können die erfindungsgemäßen (Meth)acrylsäureester der Formel (I) mit an sich bekannten Monomeren gemischt werden, um beispielsweise die Viskosität dem Verwendungszweck anzupassen. Viskositäten im Bereich von 60 bis 20.000 mPa.s sind dabei bevorzugt. Dies ist dadurch erreichbar, daß man den erfindungsgemäßen Monomeren gegebenenfalls ein Comonomer niedrigerer Viskosität zumischt. Die erfindungsgemäßen Verbindungen werden in der Mischung mit Comonomeren mit einem Anteil von ca. 10 bis 90 Gew.-% eingesetzt, wobei ein Anteil von 20 bis 80 Gew.-% besonders bevorzugt ist.

Es ist auch möglich, Monomermischungen einzusetzen, die mehrere Comonomere enthalten.

Beispielsweise seien die folgenden Comonomere genannt: Triethylenglykoldimethacrylat, Tetraethylenglykoldimethacrylat, 1,12-Dodecandioldimethacrylat, 1,6-Hexandioldimethacrylat, Tri- und Diethylenglykoldimethacrylat, Bis-GMA, 2,2-Bis[p-(2'-methacryloyloxyethoxy)phenyl]propan. Von Vorteil sind auch Comonomere mit Urethangruppen, z.B. die bekannten Umsetzungsprodukte von 1 Mol eines Diisocyanats, z.B. Hexamethylendiisocyanat, Trimethylhexamethylendiisocyanat oder Isophorondiisocyanat, mit 2 Molen eines Hydroxyalkyl(meth)acrylsäureesters, z.B. Glycerindimethacrylat, 2-Hydroxypropylacrylat usw.

Weitere Beispiele für Comonomere sind: Trimethylolpropan-tri(meth)-acrylat, Bis-(Meth)-acryloyloxyethoxymethyl-Tricyclo[5.2,1.$0^{2,6}$]decan (gemäß DE-A 2 931 925 und 2 931 926), 1,3-Di[(meth)-acryloyloxypropyl]-1,1,3,3-tetramethyl-disiloxan, 1,3-Bis[3-(meth)-acryloyloxyethylcarbamoyloxy-propyl]-1,1,3,3-tetramethyl-disiloxan. Insbesondere werden Comonomere bevorzugt, die bei 13 mbar einen Siedepunkt über 100 ° C besitzen.

Die erfindungsgemäßen (Meth)acrylsäureester (I) lassen sich, gegebenenfalls in Mischung mit den genannten Monomeren, zur Herstellung von polymeren Werkstoffen verwenden. Die Polymerisation ergibt Polymerisate, die eine hohe Vernetzungsdichte und gegenüber herkömmlichen Polymerisaten eine Erniedrigung der polaren Anteile der Oberflächenspannung aufweisen, Die erfindungsgemäßen (Meth)-acrylsäurederivate (I) können insbesondere als Monomere für Dentalmaterialien verwendet werden. Als Dentalmaterialien seien beispielsweise Füllungsmaterialien für Zähne, Beschichtungsmittel für Zähne und Komponenten für die Herstellung von Zahnersatz genannt. Je nach Anwendungsgebiet können Dentalmaterialien weitere Hilfsstoffe enthalten.

Die erfindungsgemäßen (Meth)acrylsäureester (I) lassen sich, gegebenenfalls in Mischung mit den genannten Monomeren, mit an sich bekannten Methoden zu vernetzten Polymerisaten aushärten (G.M. Brauer, H. Argentar, Am, Chem. Soc., Symp. Ser. 212, S. 359 bis 371 [1983]). Für die sogenannte Redoxpolymerisation ist ein System aus einer peroxidischen Verbindung und einem Reduktionsmittel, beispielsweise auf Basis tertiärer aromatischer Amine, geeignet.

Beispiele für Peroxide sind:
Dibenzoylperoxid, Dilauroylperoxid, Di-4-Chlorbenzolperoxid und Dicyclohexylperoxydicarbonat.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-PS 2 759 239 beschriebene N-Methyl-N-(2-methylcarbamoyloxypropyl)-3,5-dimethylanilin genannt.

Die Konzentrationen des Peroxids bzw. des Amins werden vorteilhaft so gewählt, daß sie 0,1 bis 5 Gew.-%, bevorzugt 0,5 bis 3 Gew,-%, bezogen auf die Monomermischung, betragen. Die peroxid- bzw. aminhaltigen Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethylaminobenzolsulfonsäurebisallylamid. Die Durchführung des Photopolymerisationsverfahrens ist beispielsweise in der DE-PS 3 135 115 beschrieben.

Neben den oben beschriebenen Initiatoren können den erfindungsgemäßen (Meth)acrylsäureestern an sich für diesen Einsatzzweck bekannte Lichtschutzmittel und Polymerisations-Inhibitoren zugesetzt werden. Das Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung, eingesetzt. Die Monomermischungen können ohne Zusatz von Füllstoffen als Beschichtungsmitel für Zahne (Zahnlacke) eingesetzt werden. Nach der Polymerisation erhält man einen kratzfesten Überzug auf dem Substrat.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 20.000 mPa.s besitzen, besonders vorteilhaft. Den die erfindungsgemäßen Verbindungen der Formel (I) enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-A 2 347 591), genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden [E.P. Plueddemann, Progress in Organic coatings, 11, 297 bis 308 (1983)]. Bevorzugt wird 3-Methacryloyloxypropyltrimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm, besonders bevorzugt 0,05 bis 5 µm, auf. Es kann auch vorteilhaft sein, mehrere Füllstoffe nebeneinander einzusetzen, die einen voneinander verschiedenen Teilchendurchmesser und verschiedenen Silanisierungsgrad besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet.

Der Anteil der erfindungsgemäßen (Meth)acrylsäure-Derivate in den Füllmassen beträgt im allgemeinen 5 bis 90 Gew.-%, vorzugsweise 10 bis 60 Gew.-%, bezogen auf die Füllmasse. Die Aushärtung der Zahnfüllmassen zu einem Formkörper erfolgt in der Kavität des Zahnes mit den oben genannten Methoden.

Die erfindungsgemäßen (Meth)acrylsäure-Derivate können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat, eingesetzt. Es können auch zusätzlich an sich bekannte Perlpolymeriste zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt. Die Verarbeitung ist sowohl nach Injektionsverfahren als auch Prägeverfahren möglich und erfolgt im allgemeinen nach den üblichen Herstellungsmethoden für Zähne auf Basis von Poly-(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersäuredinitril. Gut geeignet sind auch Mischungen von Polymerisationsinitiatoren mit unterschiedlichen Halbwertzeiten bezüglich ihres Zerfalls.

## Beispiele

### Beispiel 1

Umsetzung des 1,2-Bis-(4'-isocyanatophenoxy)-3,3,4,4,5,5-hexafluor-1-cyclopentens (A) mit Glycerindimethacrylat (GDMA)

Zu einer Lösung aus 88,90 g (0,201 Mol) Isocyanat A in 170 g trockenem Chloroform wurden unter Rühren 91,75 g (0,402 Mol) GDMA getropft, wobei die Temperatur auf 61 °C anstieg. Es wurde noch elf Stunden bei Raumtemperatur gerührt, mit je 0,60 g 2,6-Di-tert.butyl-p-kresol und Hydrochinonmonomethylether versetzt und zu 178,24 g (98 % d. Th.) eines hellen, viskosen Öles an Produkt "A-GDMA$_2$" (s. vorn) eingeengt.

| | |
|---|---|
| IR (Film): | $\nu$ = 3300, 2900, 1720, 1632, 1608, 1522, 1500, 1351, 1283, 1185, 1150, 1000, 973, 940, 754 cm$^{-1}$. |
| $^1$H-NMR ($CDCl_3$, 200 MHz): | $\delta$ = 1.97 (bs, 12 H, $CH_3$), 4.30 (m, 10 H, $CH_2$-CH-$CH_2$), 5.62, 6.15 (2 m, je 4 H, vinyl. H), 6.71, 7.20 (2 m, je 4 H, ar. H), 6.95 (bs, 2 H, NH) ppm. |
| HPLC/MS ($NH_3$-Aktivierung): | m/z = 898 (M$^+$), 670 (M-GDMA) |
| MS (70 eV-Direkteinlaß): | |

m/z = 584 (M-GDMA-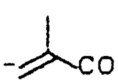$CO_2$H),

484 (M-GDMA-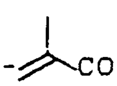$CO_2$H-$C_2F_4$),

442 (M-2 GDMA), 69 (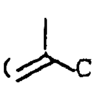C≡O$^{\oplus}$).

Beispiel 2

Umsetzung des 1,2-Bis-(4'-isocyanatophenoxy)-3,3,4,4,5,5-hexafluor-1-cyclopentens (A) mit 2-Hydroxyethylmethacrylat (HEMA)

Zu einer Lösung aus 200,00 g (452 mmol) Isocyanat A in 375,00 g trockenem Chloroform wurden unter Rühren bei Raumtemperatur 117,65 g (904 mmol) HEMA getropft und fünf Stunden lang auf 50 °C erwärmt. Nach Zugabe von je 1,71 g 2,6-Di-tert.butyl-p-kresol und Hydrochinonmonomethylether wurde der Ansatz zu 316,76 g (99 % d. Th.) eines hellen Öles an Produkt "A-HEMA$_2$" (s. vorn) eingeengt.

| | |
|---|---|
| IR (Film): | $\nu$ = 3280, 2900, 1701, 1620, 1600, 1520, 1500, 1360, 1280, 1208, 1180, 1151, 1067, 992, 963 $cm^{-1}$. |
| $^1$H-NMR ($CDCl_3$, 200 MHz) | : $\delta$ = 1.97 (bs, 6 H, $CH_3$), 4.42 (bs, 8 H, $CH_2$), 5.61, 6.17 (2 m, je 2 H, vinyl. H), 6.67, 7.16 (2 m, je 4 H, ar. H), 6.71 (bs, 2 H, NH) ppm. |
| MS (70 eV): | m/z = 702 ($M^+$), 572 (M-HEMA), 442 (M-2HEMA), |

113 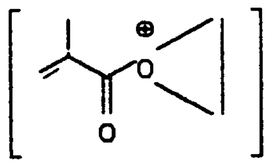 , 69 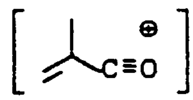 .

Beispiel 3

Umsetzung des 1,2-Bis-[4'-(chlorocarbonyl)phenoxy]-3,3,4,4,5,5-hexafluor-1-cyclopentene (B) mit 2-Hydroxyethylmethacrylat (HEMA)

65,20 g (500 mmol) HEMA, 60,80 g (600 mmol) trockenes Triethylamin und 50 mg 2,6-Di-tert.butyl-kresol wurden zusammen in 250 ml trockenem Methylenchlorid vorgelegt und bei -30 °C mit einer Lösung aus 121,30 g (250 mmol) Säurechlorid B in 200 ml Methylenchlorid versetzt. Es wurde zwei Stunden bei -30 °C gerührt, der ausgefallene Niederschlag bei 0 °C abgesaugt und das erhaltene Filtrat wäßrig extrahiert. Die getrocknete organische Phase wurde mit 50 mg Hydrochinonmonomethylether versetzt und zu 157,20 g (93 % d. Th.) eines hellen Öles an Produkt "B-HEMA$_2$" (s. vorn) eingeengt.

| | |
|---|---|
| IR (Film): | $\nu$ = 2940, 1720, 1618, 1602, 1500, 1445, 1270, 1150, 1002, 978, 940, 845, 809, 760 $cm^{-1}$. |
| $^1$H-NMR ($CDCl_3$, 200 MHz): | $\delta$ = 1.96 (bs, 6 H, $CH_3$), 4.50 (m, 8 H, $CH_2$), 5.61, 6.13 (2 m, je 2 H, vinyl. H), 6.78, 7.82 (2 m, je 4 H, ar. H) ppm. |

Beispiel 4

Umsetzung des 1,2-Bis-[4'-(chlorocarbonyl)phenoxy]-3,3,4,4,5,5-hexafluor-1-cyclopentens (B) mit Glycerindimethacrylat (GDMA)

114,00 g (500 mmol) Glycerindimethacrylat, 60,80 g (600 mmol) trockenes Triethylamin und 50 mg 2,6-Di-tert.butyl-p-kresol wurden zusammen in 250 ml trockenem Methylenchlorid vorgelegt und bei -30 °C mit einer Lösung aus 121,30 g (250 mmol) Säurechlorid B in 250 ml Methylenchlorid versetzt. Es wurde nun wie in Beispiel 3 verfahren und 205,56 g (94 % d. Th.) eines hellen Öles an Produkt "B-GDMA$_2$" (s. vorn) erhalten.

| | |
|---|---|
| IR (Film): | $\nu$ = 2960, 1730, 1640, 1605, 1500, 1350, 1321, 1295, 1267, 1205, 1157, 1110, 1005, 979, 942, 847, 810, 759, 730 $cm^{-1}$. |
| $^1$H-NMR ($CDCl_3$, 200 MHz): | $\delta$ = 1.93 (bs, 12 H, $CH_3$), 4.40 (m, 10 H, $CH_2$-CH-$CH_2$), 5.61, 6.11 (2 m, je 4 H, vinyl. H), 6.77, 7.86 (2 m, je 4 H, ar. H) ppm. |

Beispiel 5

Umsetzung des 1,2-Bis-[4'-(Isocyanato-2'-trifluormethylphenoxy)-3,3,4,4,5,5-hexafluor-1-cyclopentens (C) mit 2-Hydroxypropylmethacrylat (HPMA) und Glycerindimethacrylat (GDMA)

Zu einer Lösung aus 57,83 g (100 mmol) Isocyanat C in 120 ml trockenem Chloroform wurde unter Rühren bei Raumtemperatur eine Lösung aus 14,42 g (100 mmol) HPMA und 22,82 g (100 mmol) GDMA in 10 ml trockenem Chloroform getropft. Es wurde vier Stunden lang auf 40 °C erwärmt und anschließend zu 91,10 g (96 % der Theorie) eines gelblichen Öles eingeengt, das aus den drei Reaktionsprodukten "C-GDMA-HPMA", "C-$HPMA_2$" und "C-$GDMA_2$" (s. vorn) bestand.

IR (Film): $\nu$ = 3300, 2940, 1710, 1620, 1540, 1492, 1420, 1320, 1200, 1130, 1044, 1000, 973, 940, 752 $cm^{-1}$.

$^1$H-NMR ($CDCl_3$, 200 MHz): $\delta$ = 1.3 (OCH-$CH_3$), 1.98 (=$CCH_3$), 3.6-4.6 ($OCH_2$, OCH), 5.6, 6.15 (= $CH_2$), 6.8-7.7 (ar.-H, NH) ppm.

Beispiel 6

3 Teile des erfindungsgemäßen (Meth)acrylsäureesters nach Beispiel 1 und 1 Teil Triethylenglykoldimethacrylat werden versetzt mit 0,2 Gew.-% Campherchinon und 0,5 Gew.-% 4-N,N-Dimethylaminobenzolsulfonsäurebisallylamid und unter Lichtausschluß zu einer aktivierten Monomermischung (D) verarbeitet. Diese härtet durch sichtbares Licht bei einer Belichtungsdauer von 60 s zu einem Kunststoff hoher mechanischer Stabilität aus und ist als Verschlußmaterial im Dentalbereich (Sealer, Liner, Zahnlack) verwendbar.

Zur Herstellung einer Zahnfüllmasse werden 29 Gewichtsteile der aktivierten nicht polymerisierten Monomermischung und 71 Gewichtsteile einer von mit 3-Methacryloyloxypropyltrimethoxysilan silanisierten Mischung aus pyrogener Kieselsäure und gemahlenem Quarzglas in einem handelsüblichen Kneter bei Raumtemperatur zu einer Paste verarbeitet. Ein nach DIN 13 922 mit einer handelsüblichen Dentallampe (Translux®) ausgehärteter Probekörper, der aus dieser Paste hergestellt wurde, zeigt neben einer hohen Biegefestigkeit und geringen Wasseraufnahme auch eine gute Abrasionsbeständigkeit.

**Patentansprüche**

**Patentansprüche für folgende Vertragsstaaten : AT, BE, CH, DE, DK, FR, GB, GR, IT, LI, LU, NL, SE**

**1.** (Meth)acrylsäureester substituierter 1,2-Bis(phenoxy)-3,3,4,4,5,5-hexafluor-1-cyclopentene der Formel

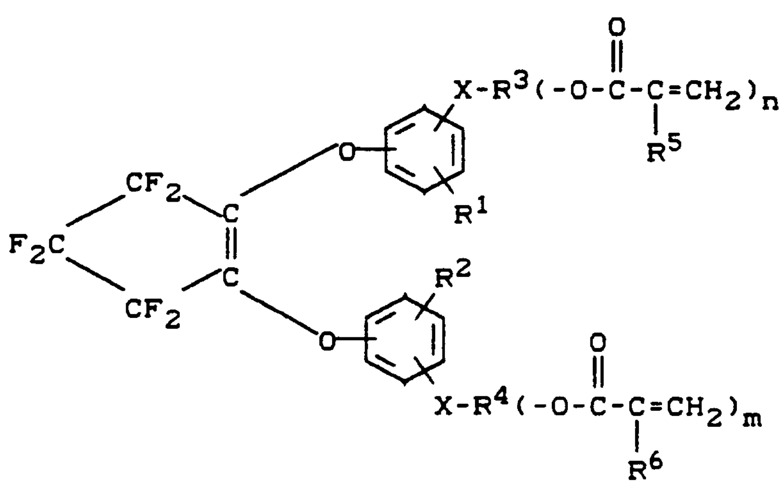

(I)

in der jeweils unabhängig voneinander

$R^1$, $R^2$ $C_1$- bis $C_4$-Alkyl, $C_1$- bis $C_4$-Halogenalkyl oder Wasserstoff bedeuten,

$R^3$, $R^4$ (n + 1)- bzw. (m + 1)-wertige, geradkettige oder verzweigte Kohlenwasserstoffreste mit 2 bis 15 Kohlenstoffstomen, die gegebenenfalls 1 bis 3 Sauerstoffbrücken enthalten können, bedeuten,

| | |
|---|---|
| $R^5$, $R^6$ | Wasserstoff oder Methyl bedeuten und |
| X | für |

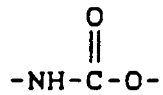 oder 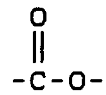

| | |
|---|---|
| | steht und |
| n und m | ganze Zahlen von 1 bis 5 bedeuten. |

**2.** (Meth)acrylsäureester substituierter 1,2-Bis(phenoxy)-3,3,4,4,5,5-hexafluor-1-cyclopentene nach Anspruch 1, wobei jeweils unabhängig voneinander

| | |
|---|---|
| $R^1$, $R^2$ | Wasserstoff oder Trifluormethyl bedeuten, |
| $R^3$, $R^4$ | (n + 1)- bzw. (m + 1)-wertige, geradkettige oder verzweigte Kohlenwasserstoffreste mit 2 bis 15 Kohlenstoffatomen bedeuten, |
| $R^5$, $R^6$ | Wasserstoff oder Methyl bedeuten und |
| X | für |

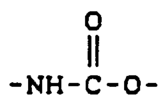 oder 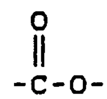

| | |
|---|---|
| | steht und |
| n und m | 1 oder 2 bedeuten. |

**3.** Ein Verfahren zur Herstellung der (Meth)acrylsäureester nach den Ansprüchen 1 und 2, dadurch gekennzeichnet, daß man substituierte 1,2-Bis(phenoxy)-3,3,4,4,5,5-hexafluor-1-cyclopentene der Formel

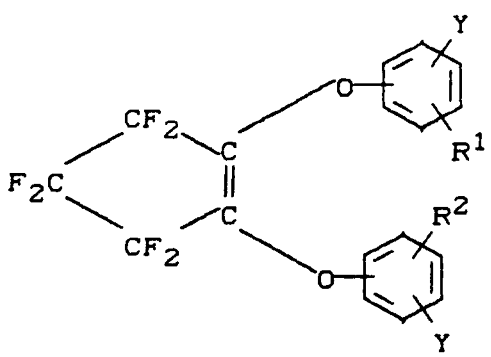

(II)

worin

| | |
|---|---|
| $R^1$, $R^2$ | unabhängig voneinander $C_1$- bis $C_4$-Alkyl, $C_1$- bis $C_4$-Halogenalkyl oder Wasserstoff bedeuten und |
| Y | für Isocyanato (-NCO) oder Chlorocarbonyl (-COCl) steht, |

mit mindestens einem OH-funktionellen (Meth)acrylsäure-Derivat der Formel

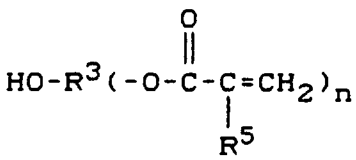 oder 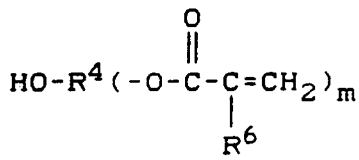

(III) (IV)

worin unabhängig voneinander

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten,

$R^5$, $R^6$ Wasserstoff oder Methyl bedeuten und

n und m ganze Zahlen von 1 bis 5 bedeuten

in einem inerten Lösungsmittel, im Temperaturbereich zwischen -40 und +100 °C, gegebenenfalls in Gegenwart eines Katalysators und/oder einer Base sowie gegebenenfalls in Gegenwart eines Polymerisationsinhibitors umsetzt.

4. Polymerisat, hergestellt aus Monomeren enthaltend (Meth)acrylsäureester substituierter 1,2-Bis-(phenoxy)-3,3,4,4,5,5-hexafluor-1-cyclopentene der Formel

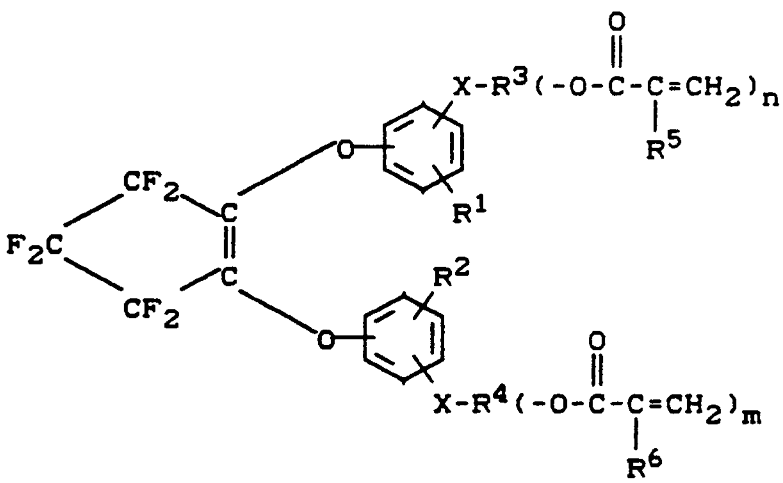

(I)

in der jeweils unabhängig voneinander

$R^1$, $R^2$ $C_1$- bis $C_4$-Alkyl, $C_1$- bis $C_4$-Halogenalkyl oder Wasserstoff bedeuten,

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten,

$R^5$, $R^6$ Wasserstoff oder Methyl bedeuten und

X für

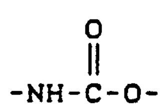 oder 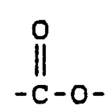

steht und

n und m ganze Zahlen von 1 bis 5 bedeuten.

**5.** Verwendung von (Meth)acrylsäureestern substituierter 1,2-Bis(phenoxy)-3,3,4,4,5,5-hexafluor-1-cyclopentene der Formel

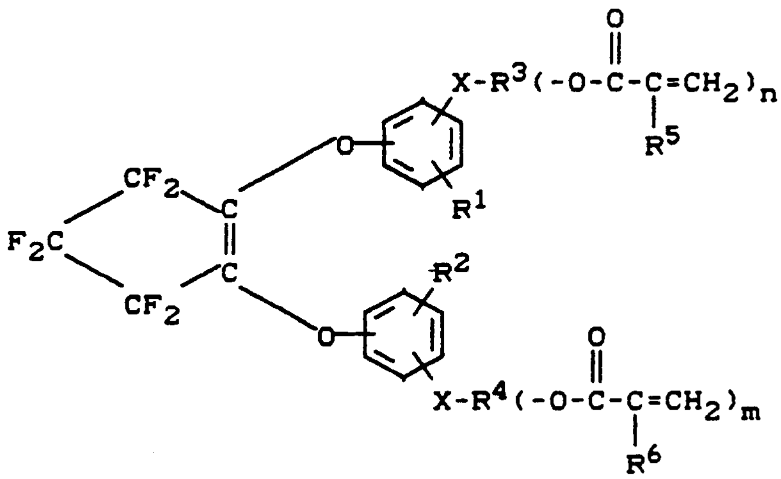

(I)

in der jeweils unabhängig voneinander

| | |
|---|---|
| $R^1$, $R^2$ | $C_1$- bis $C_4$-Alkyl, $C_1$- bis $C_4$-Halogenalkyl oder Wasserstoff bedeuten, |
| $R^3$, $R^4$ | (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten, |
| $R^5$, $R^6$ | Wasserstoff oder Methyl bedeuten und |
| X | für |

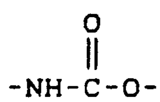 oder 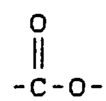

| | |
|---|---|
| | steht und |
| n und m | ganze Zahlen von 1 bis 5 bedeuten, im Dentalbereich. |

**6.** Verwendung nach Anspruch 5, dadurch gekennzeichnet, daß die (Meth)acrylsäureester substituierter 1,2-bis(phenoxy)-3,3,4,4,5,5-hexafluor-1-cyclopentene in Zahnfüllmassen eingesetzt werden.

**7.** Verwendung nach Anspruch 5, dadurch gekennzeichnet, daß die (Meth)acrylsäureester substituierter 1,2-Bis(phenoxy)-3,3,4,4,5,5-hexafluor-1-cyclopentene in Beschichtungsmitteln für Zähne eingesetzt werden.

**8.** Verwendung nach Anspruch 5, dadurch gekennzeichnet, daß die (Meth)acrylsäureester substituierter 1,2-Bis(phenoxy)-3,3,4,4,5,5-hexafluor-1-cyclopentene für die Herstellung von Kunststoffzähnen eingesetzt werden.

**9.** Härtbare Dentalmaterialien, dadurch gekennzeichnet, daß sie (Meth)acrylsäureester substituierter 1,2-Bis(phenoxy)-3,3,4,4,5,5-hexafluor-1-cyclopentene der Formel

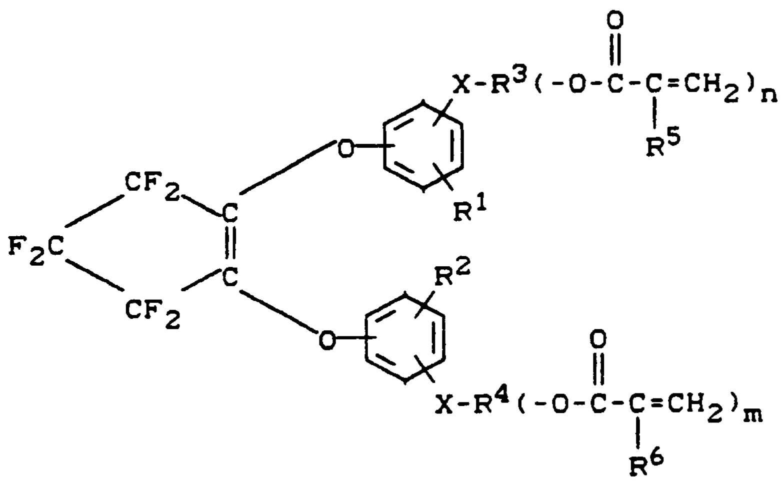

(I)

in der jeweils unabhängig voneinander

| | |
|---|---|
| $R^1$, $R^2$ | $C_1$- bis $C_4$-Alkyl, $C_1$- bis $C_4$-Halogenalkyl oder Wasserstoff bedeuten, |
| $R^3$, $R^4$ | (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten, |
| $R^5$, $R^6$ | Wasserstoff oder Methyl bedeuten und |
| X | für |

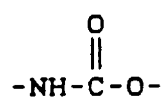 oder 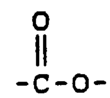

| | |
|---|---|
| | steht und |
| n und m | ganze Zahlen von 1 bis 5 bedeuten, enthalten. |

**Patentansprüche für folgenden Vertragsstaat : ES**

1. Verfahren zur Herstellung von (Meth)acrylsäureestern substituierter 1,2-Bis-(phenoxy)-3,3,4,4,5,5-hexafluor-1-cyclopentene der Formel (I)

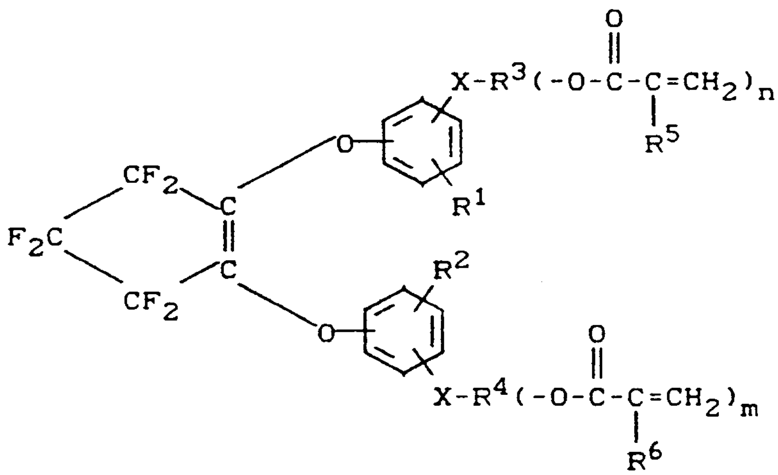

(I)

in der jeweils unabhängig voneinander

$R^1$, $R^2$ $C_1$- bis $C_4$-Alkyl, $C_1$- bis $C_4$-Halogenalkyl oder Wasserstoff bedeuten,

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten,

$R^5$, $R^6$ Wasserstoff oder Methyl bedeuten und

X für

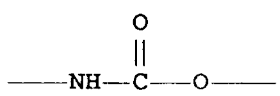 oder 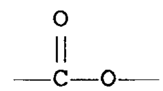

steht und

n und m ganze Zahlen von 1 bis 5 bedeuten,

dadurch gekennzeichnet, daß man 1,2-Bis(phenoxy)-3,3,4,4,5,5-hexaflour-1-cyclopentene der Formel (II)

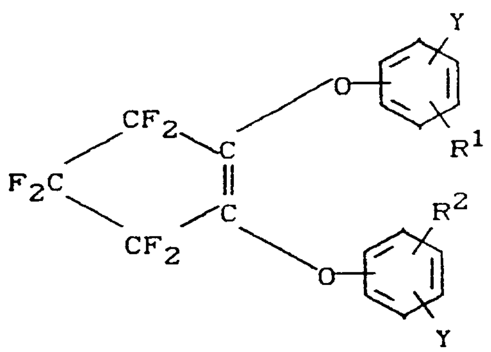

(II)

worin

$R^1$, $R^2$ unabhängig voneinander $C_1$- bis $C_4$-Alkyl, $C_1$- bis $C_4$-Halogenalkyl oder Wasserstoff bedeuten und

Y für Isocyanato (-NCO) oder Chlorocarbonyl (-COCl) steht,

mit mindestens einem OH-funktionellen (Meth)acrylsäure-Derivat der Formel

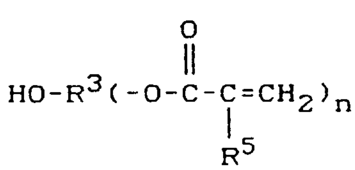

(III)

oder

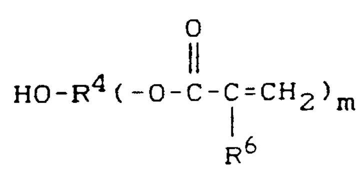

(IV)

worin unabhängig voneinander

$R^3$, $R^4$ (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten,

$R^5$, $R^6$ Wasserstoff oder Methyl bedeuten und

n und m ganze Zahlen von 1 bis 5 bedeuten

in einem inerten Lösungsmittel, im Temperaturbereich zwischen -40 und +100 °C, gegebenenfalls in Gegenwart eines Katalysators und/oder einer Base sowie gegebenenfalls in Gegenwart eines Polymerisationsinhibitors umsetzt.

**2.** Verwendung von (Meth)acrylsäureestern substituierter 1,2-Bis(phenoxy)-3,3,4,4,5,5-hexafluor-1-cyclopentene der Formel

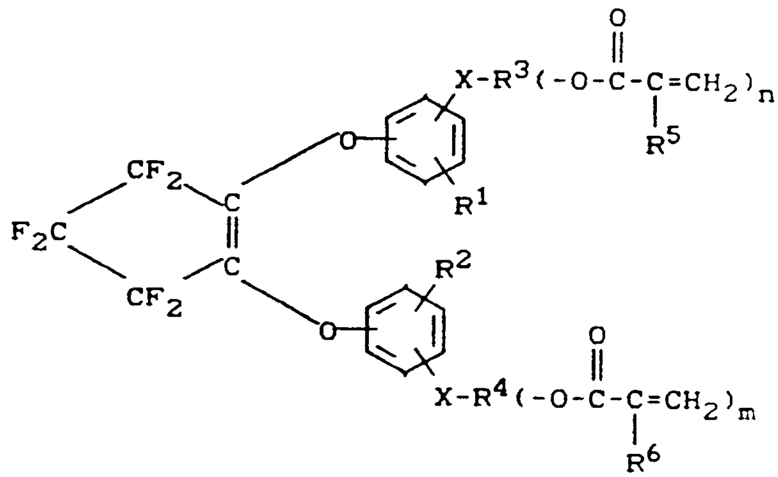

(I)

in der jeweils unabhängig voneinander

| | |
|---|---|
| $R^1$, $R^2$ | $C_1$- bis $C_4$-Alkyl, $C_1$- bis $C_4$-Halogenalkyl oder Wasserstoff bedeuten, |
| $R^3$, $R^4$ | (n + 1)- bzw. (m + 1)-wertige, geradkettige oder verzweigte Kohlenwasserstoffreste mit 2 bis 15 Kohlenstoffatomen, die gegebenenfalls 1 bis 3 Sauerstoffbrücken enthalten können, bedeuten, |
| $R^5$, $R^6$ | Wasserstoff oder Methyl bedeuten und |
| X | für |

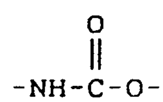

oder

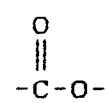

steht und

| | |
|---|---|
| n und m | ganze Zahlen von 1 bis 5 bedeuten, im Dentalbereich. |

**3.** Verwendung nach Anspruch 2, dadurch gekennzeichnet, daß die (Meth)acrylsäureester substituierter 1,2-bis(phenoxy)-3,3,4,4,5,5-hexafluor-1-cyclopentene in Zahnfüllmassen eingesetzt werden.

**4.** Verwendung nach Anspruch 2, dadurch gekennzeichnet, daß die (Meth)acrylsäureester substituierter 1,2-Bis(phenoxy)-3,3,4,4,5,5-hexafluor-1-cyclopentene in Beschichtungsmitteln für Zähne eingesetzt werden.

**5.** Verwendung nach Anspruch 2, dadurch gekennzeichnet, daß die (Meth)acrylsäureester substituierter 1,2-Bis(phenoxy)-3,3,4,4,5,5-hexafluor-1-cyclopentene für die Herstellung von Kunststoffzähnen eingesetzt werden.

**Claims**

**Claims for the following Contracting States : AT, BE, CH, DE, DK, FR, GB, GR, IT, LI, LU, NL, SE**

**1.** (Meth)acrylic acid esters of substituted 1,2-bis-(phenoxy)-3,3,4,4,5,5-hexafluoro-1-cyclopentenes of the formula

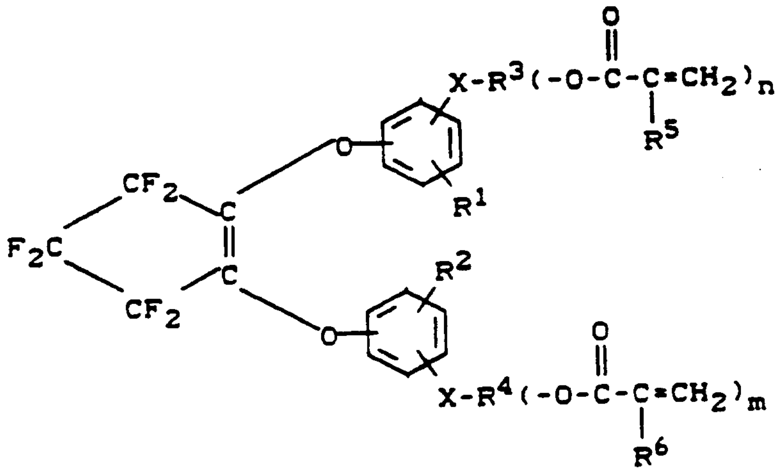

(I)

in which, in each case independently of one another,

- $R^1$ and $R^2$ denote $C_1$- to $C_4$-alkyl, $C_1$- to $C_4$-halogenoalkyl or hydrogen,
- $R^3$ and $R^4$ denote (n + 1)- or (m + 1)-valent, straight-chain or branched hydro-carbon radicals having 2 to 15 carbon atoms, which may optionally contain 1 to 3 oxygen bridges,
- $R^5$ and $R^6$ denote hydrogen or methyl and
- X represents

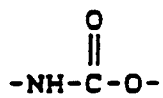

or

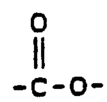

and

- n and m denote integers from 1 to 5.

**2.** (Meth)acrylic acid esters of substituted 1,2-bis-(phenoxy)-3,3,4,4,5,5-hexafluoro-1-cyclopentenes according to Claim 1, where, in each case independently of one another,

- $R^1$ and $R^2$ denote hydrogen or trifluoromethyl,
- $R^3$ and $R^4$ denote (n + 1)- or (m + 1)-valent, straight-chain or branched hydro-carbon radicals having 2 to 15 carbon atoms,
- $R^5$ and $R^6$ denote hydrogen or methyl and
- X represents

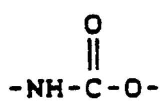

or

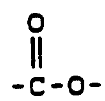

and

- n and m denote 1 or 2.

**3.** Process for the preparation of the (meth)acrylic acid esters according to Claims 1 and 2, characterized in that substituted 1,2-bis(phenoxy)-3,3,4,4,5,5-hexafluoro-1-cyclopentenes of the formula

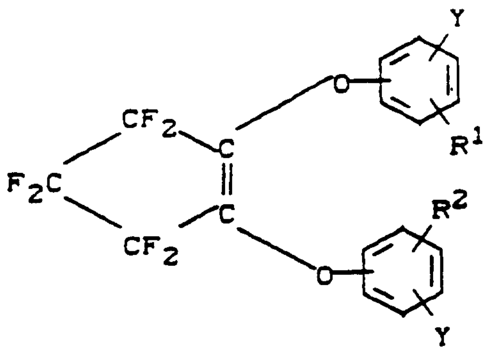

(II)

in which

$R^1$ and $R^2$ independently of one another denote $C_1$- to $C_4$-alkyl, $C_1$- to $C_4$-halogenoalkyl or hydrogen and

Y represents isocyanato (-NCO) or chlorocarbonyl (-COCl),

are reacted with at least one OH-functional (meth) - acrylic acid derivative of the formula

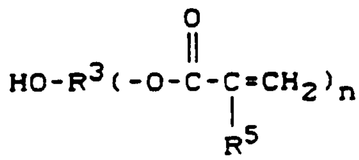

or

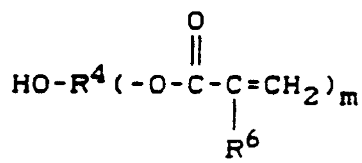

(III) (IV)

in which, independently of one another,

$R^3$ and $R^4$ denote (n + 1)- or (m + 1)-valent, straight-chain or branched hydro-carbon radicals having 2 to 15 carbon atoms, which may optionally contain 1 to 3 oxygen bridges,

$R^5$ and $R^6$ denote hydrogen or methyl and

n and m denote integers from 1 to 5

in an inert solvent, in the temperature range between -40 and +100 °C, optionally in the presence of a catalyst and/or a base and optionally in the presence of a polymerization inhibitor.

**4.** Polymer, prepared from monomers containing (meth)--acrylic acid esters of substituted 1,2-bis-(phenoxy)-3,3,4,4,5,5-hexafluoro-1-cyclopentenes of the formula

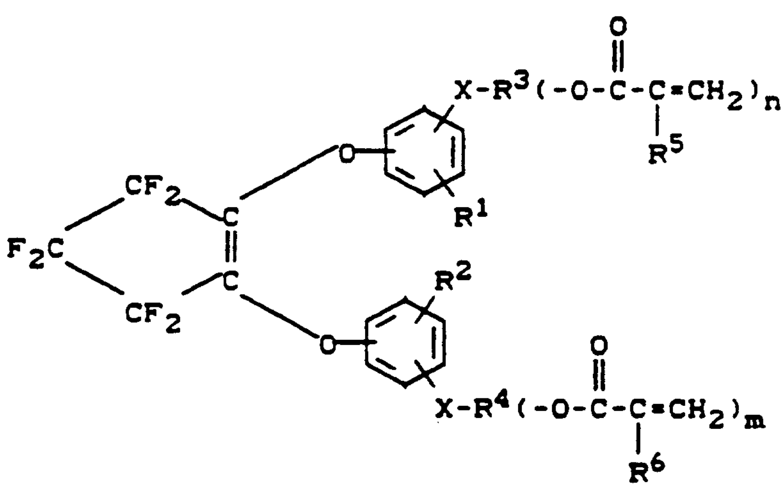

(I)

in which, in each case independently of one another,

R[1] and R[2] denote $C_1$- to $C_4$-alkyl, $C_1$- to $C_4$-halogenoalkyl or hydrogen,

R[3] and R[4] denote (n + 1)- or (m + 1)-valent, straight-chain or branched hydro-carbon radicals having 2 to 15 carbon atoms, which may optionally contain 1 to 3 oxygen bridges,

R[5] and R[6] denote hydrogen or methyl and

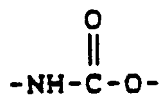

or

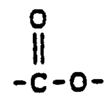

X represents and

n and m denote integers from 1 to 5.

**5.** Use of (meth)acrylic acid esters of substituted 1,2-bis(phenoxy)-3,3,4,4,5,5-hexafluoro-1-cyclopentenes of the formula

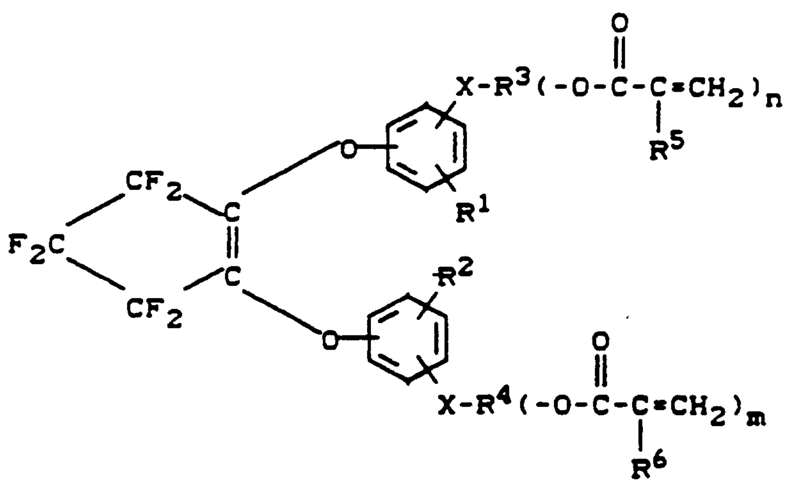

(I)

in which, in each case independently of one another,

R[1] and R[2] denote $C_1$- to $C_4$-alkyl, $C_1$- to $C_4$-halogenoalkyl or hydrogen,

R[3] and R[4] denote (n + 1)- or (m + 1)-valent, straight-chain or branched hydro-carbon radicals having 2 to 15 carbon atoms, which may optionally contain 1 to 3 oxygen bridges,

R[5] and R[6] denote hydrogen or methyl and

X represents

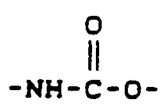

or

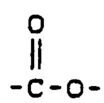

and

n and m denote integers from 1 to 5, in the dental field.

**6.** Use according to Claim 5, characterized in that the (meth)acrylic acid esters of substituted 1,2-bis-(phenoxy)-3,3,4,4,5,5-hexafluoro-1-cyclopentenes are employed in dental filling materials.

**7.** Use according to Claim 5, characterized in that the (meth)acrylic acid esters of substituted 1,2,-bis-(phenoxy)-3,3,4,4,5,5-hexafluoro-1-cyclopentenes are employed in coating agents for teeth.

8. Use according to Claim 5, characterized in that the (meth)acrylic acid esters of substituted 1,2-bis-(phenoxy)-3,3,4,4,5,5-hexafluoro-1-cyclopentenes are employed for the preparation of artificial teeth.

9. Curable dental materials, characterized in that they contain (meth)acrylic acid esters of substituted 1,2-bis(phenoxy)-3,3,4,4,5,5-hexafluoro-1-cyclopentenes of the formula

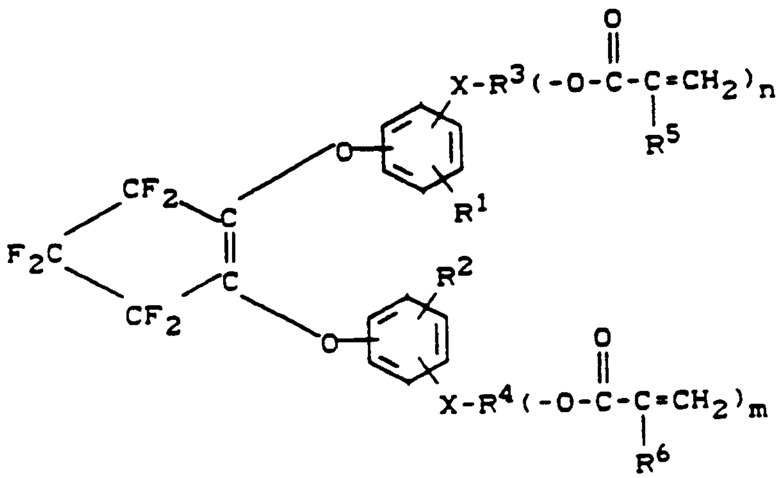

(I)

in which, in each case independently of one another,

| | |
|---|---|
| $R^1$ and $R^2$ denote | $C_1$- to $C_4$-alkyl, $C_1$- to $C_4$-halogenoalkyl or hydrogen, |
| $R^3$ and $R^4$ denote | (n + 1)- or (m + 1)-valent, straight-chain or branched hydro-carbon radicals having 2 to 15 carbon atoms, which may optionally contain 1 to 3 oxygen bridges, |
| $R^5$ and $R^6$ denote | hydrogen or methyl and |
| X represents | |

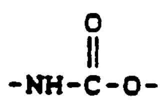

or

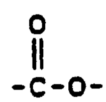

and

n and m denot e integers from 1 to 5.

**Claims for the following Contracting State : ES**

1. Process for the preparation of (meth)acrylic acid esters of substituted 1,2-bis-(phenoxy)-3,3,4,4,5,5-hexafluoro-1-cyclopentenes of the formula (I)

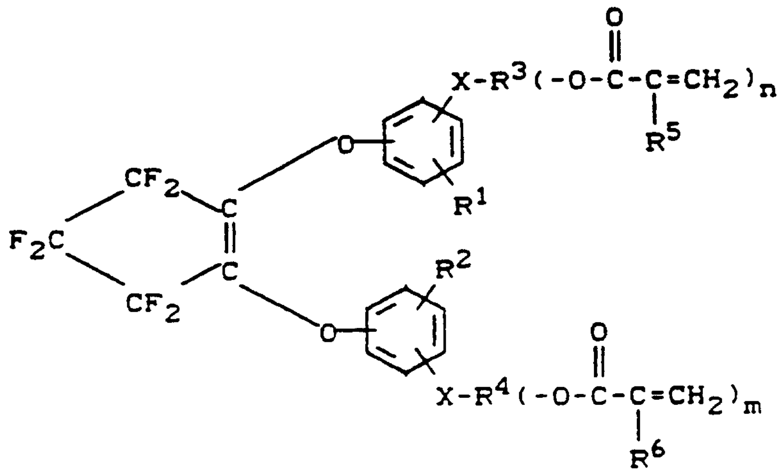

(I)

in which, in each case independently of one another,

$R^1$ and $R^2$ denote $C_1$- to $C_4$-alkyl, $C_1$- to $C_4$-halogenoalkyl or hydrogen,

$R^3$ and $R^4$ denote (n + 1)- or (m + 1)-valent, straight-chain or branched hydro-carbon radicals having 2 to 15 carbon atoms, which may optionally contain 1 to 3 oxygen bridges,

$R^5$ and $R^6$ denote hydrogen or methyl and

X represents

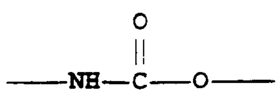 or 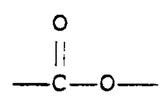

and

n and m denote integers from 1 to 5, characterized in that 1,2-bis-(phenoxy)-3,3,4,4,5,5-hexafluoro-1-cyclopentenes of the formula (II)

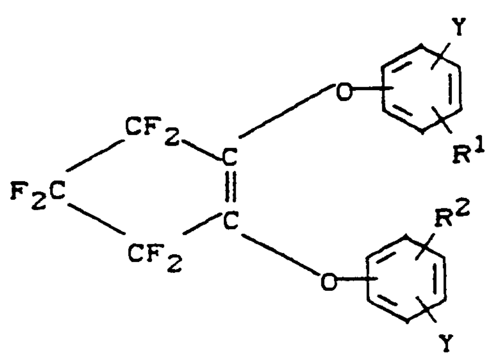

(II)

in which

$R^1$ and $R^2$ independently of one another denote $C_1$- to $C_4$-alkyl, $C_1$- to $C_4$-halogenoalkyl or hydrogen and

Y represents isocyanato (-NCO) or chlorocarbonyl (-COCl),

are reacted with at least one OH-functional (meth)-acrylic acid derivative of the formula

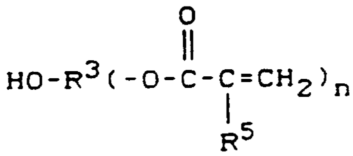

or

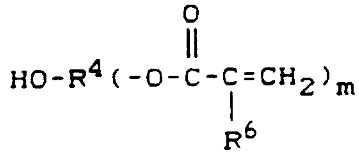

(III) (IV)

in which, independently of one another,

| | |
|---|---|
| $R^3$ and $R^4$ denote | (n + 1)- or (m + 1)-valent, straight-chain or branched hydro-carbon radicals having 2 to 15 carbon atoms, which may optionally contain 1 to 3 oxygen bridges, |
| $R^5$ and $R^6$ denote | hydrogen or methyl and |
| n and m denote | integers from 1 to 5 |

in an inert solvent, in the temperature range between -40 and +100 °C, optionally in the presence of a catalyst and/or a base and optionally in the presence of a polymerization inhibitor.

**2.** Use of (meth)acrylic acid esters of substituted 1,2-bis(phenoxy)-3,3,4,4,5,5-hexafluoro-1-cyclopentenes of the formula

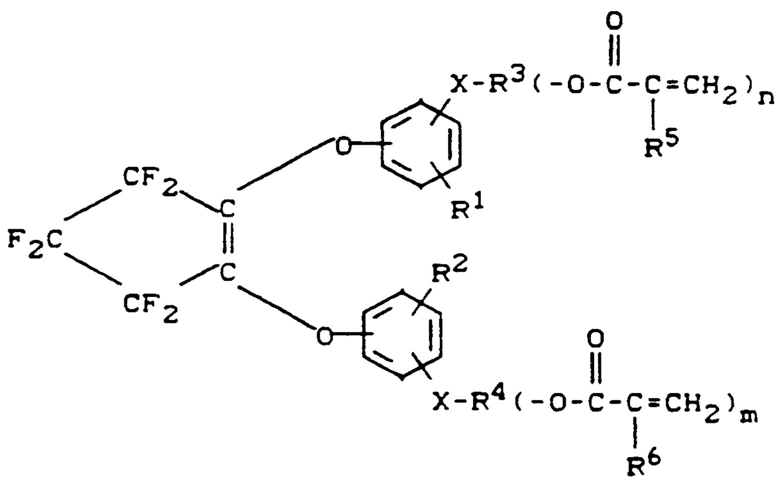

(I)

in which, in each case independently of one another,

| | |
|---|---|
| $R^1$ and $R^2$ denote | $C_1$- to $C_4$-alkyl, $C_1$- to $C_4$-halogenoalkyl or hydrogen, |
| $R^3$ and $R^4$ denote | (n + 1)- or (m + 1)-valent, straight-chain or branched hydro-carbon radicals having 2 to 15 carbon atoms, which may optionally contain 1 to 3 oxygen bridges, |
| $R^5$ and $R^6$ denote | hydrogen or methyl and |
| X represents | |

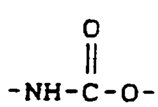

or

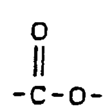

and

| | |
|---|---|
| n and m denote | integers from 1 to 5, in the dental field. |

**3.** Use according to Claim 2, characterized in that the (meth)acrylic acid esters of substituted 1,2-bis-(phenoxy)-3,3,4,4,5,5-hexafluoro-1-cyclopentenes are employed in dental filling materials.

4. Use according to Claim 2, characterized in that the (meth)acrylic acid esters of substituted 1,2,-bis-(phenoxy)-3,3,4,4,5,5-hexafluoro-1-cyclopentenes are employed in coating agents for teeth.

5. Use according to Claim 2, characterized in that the (meth)acrylic acid esters of substituted 1,2-bis-(phenoxy)-3,3,4,4,5,5-hexafluoro-1-cyclopentenes are employed for the preparation of artificial teeth.

**Revendications**

**Revendications pour les Etats contractants suivants : AT, BE, CH, DE, DK, FR, GB, GR, IT, LI, LU, NL, SE**

1. Esters d'acide (méth)acrylique de 1,2-bis-(phénoxy)-3,3,4,4,5,5-hexafluoro-1-cyclopentènes substitués de formule

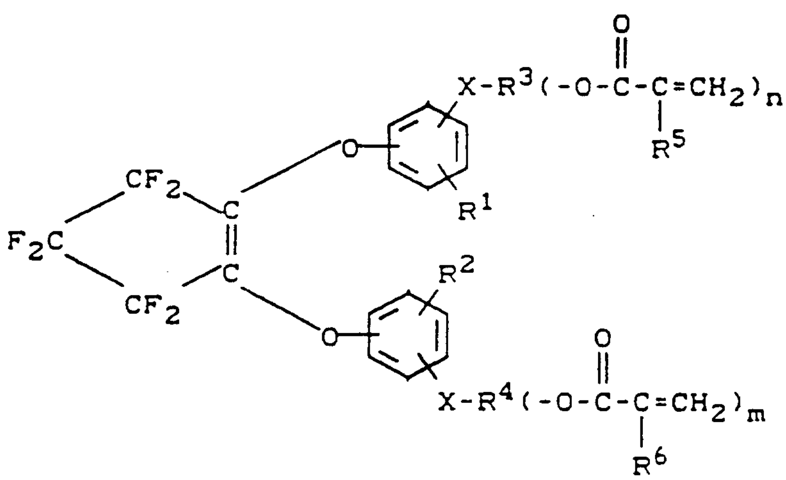

(I)

dans laquelle, indépendamment les uns des autres,

| | |
|---|---|
| $R^1$, $R^2$ | représentent un groupe alkyle en $C_1$ à $C_4$, un groupe halogénalkyle en $C_1$ à $C_4$ ou de l'hydrogène, |
| $R^3$, $R^4$ | représentent des restes hydrocarbonés à chaîne droite ou ramifies de valence (n + 1) ou (m + 1), ayant 2 à 15 atomes de carbone, qui peuvent contenir le cas échéant 1 à 3 ponts oxygène, |
| $R^5$, $R^6$ | représentent de l'hydrogène ou un groupe méthyle et |
| X | représente |

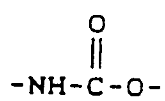

ou

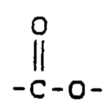

et

| | |
|---|---|
| n et m | sont des nombres entiers de 1 à 5. |

2. Esters d'acide (méth)acrylique de 1,2-bis-(phénoxy)-3,3,4,4,5,5-hexafluoro-1-cyclopentènes substitués suivant la revendication 1, dans lesquels, indépendamment les uns des autres,

| | |
|---|---|
| $R^1$, $R^2$ | représentent de l'hydrogène ou un groupe trifluorométhyle, |
| $R^3$, $R^4$ | sont des restes hydrocarbonés à chaîne droite ou ramifiés de valence (n + 1) ou (m + 1), ayant 2 à 15 atomes de carbone, |
| $R^5$, $R^6$ | représentent de l'hydrogène ou un groupe méthyle et |
| X | représente |

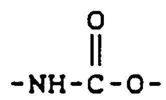 ou 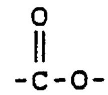

et

n et m ont la valeur 1 ou 2.

**3.** Procédé de production des esters d'acide (méth)acrylique suivant les revendications 1 et 2, caractérisé en ce qu'on fait réagir des 1,2-bis(phénoxy)-3,3,4,4,5,5-hexafluoro-1-cyclopentènes substitués de formule

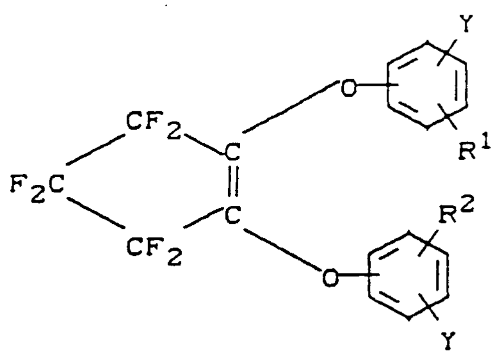

(II)

dans laquelle

$R^1$, $R^2$ représentent indépendamment l'un de l'autre un groupe alkyle en $C_1$ à $C_4$, un groupe halogénalkyle en $C_1$ à $C_4$ ou de l'hydrogène et

Y est un groupe isocyanato (-NCO) ou chlorocarbonyle (-COCl),

avec au moins un dérivé d'acide (méth)acrylique à fonctionnalité OH, de formule

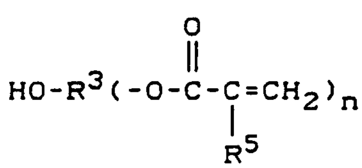

(III)

ou

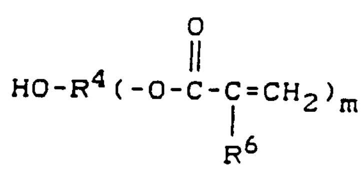

(IV)

dans laquelle, indépendamment les uns des autres,

$R^3$, $R^4$ sont des restes hydrocarbonés à chaîne droite ou ramifiée de valence (n + 1) ou (m + 1) ayant 2 à 15 atomes de carbone, qui peuvent contenir le cas échéant 1 à 3 ponts oxygène,

$R^5$, $R^6$ représentent de l'hydrogène ou un groupe méthyle et

n et m sont des nombres entiers de 1 à 5, dans un solvant inerte, dans une plage de températures de -40 à +100 °C, le cas échéant en présence d'un catalyseur et/ou d'une base ainsi que, le cas échéant, en présence d'un inhibiteur de polymérisation.

**4.** Polymère préparé à partir de monomères contenant des esters d'acide (méth)acrylique de 1,2-bis-(phénoxy)-3,3,4,4,5,5-hexafluoro-1-cyclopentènes substitués de formule

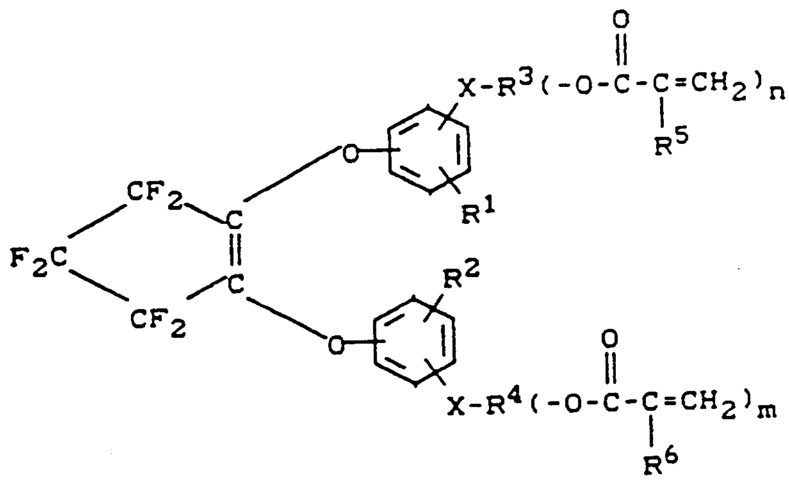

(I)

dans laquelle, indépendamment les uns des autres,

$R^1$, $R^2$ représentent un groupe alkyle en $C_1$ à $C_4$, un groupe halogénalkyle en $C_1$ à $C_4$ ou de l'hydrogène,

$R^3$, $R^4$ représentent des restes hydrocarbonés à chaîne droite ou ramifiés de valence (n + 1) ou (m + 1) ayant 2 à 15 atomes de carbone, qui peuvent contenir le cas échéant 1 à 3 ponts oxygène,

$R^5$, $R^6$ représentent de l'hydrogène ou un groupe méthyle et

X représente

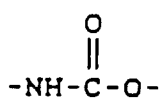 ou 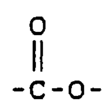

et

n et m sont des nombres entiers de 1 à 5.

**5.** Utilisation d'esters d'acide (méth)acrylique de 1,2-bis(phénoxy)-3,3,4,4,5,5-hexafluoro-1-cyclopentènes substitués de formule

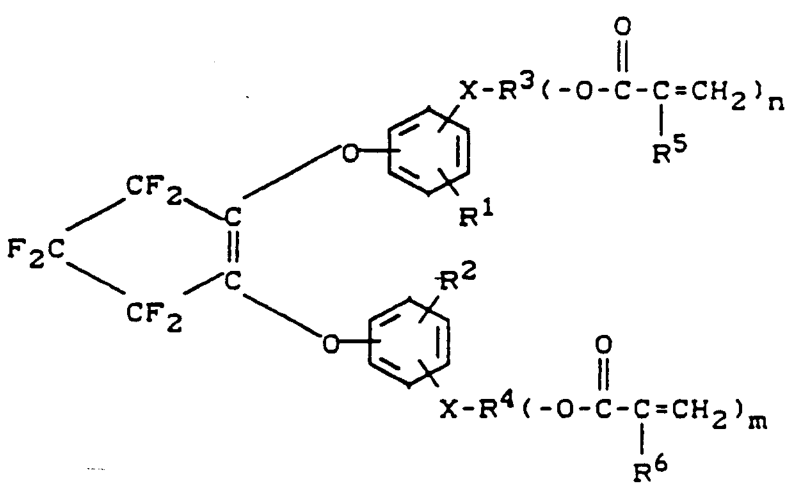

(I)

dans laquelle, indépendamment les uns des autres,

$R^1$, $R^2$ représentent un groupe alkyle en $C_1$ à $C_4$, un groupe halogénalkyle en $C_1$ à $C_4$ ou de l'hydrogène,

$R^3$, $R^4$ représentent des restes hydrocarbonés à chaîne droite ou ramifiée de valence (n + 1) ou

(m + 1), ayant 2 à 15 atomes de carbone, qui peuvent contenir le cas échéant 1 à 3 ponts oxygène,

$R^5$, $R^6$ représentent de l'hydrogène ou un groupe méthyle et

X représente

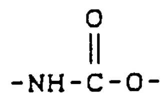

ou

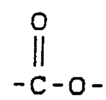

et

n et m sont des nombres entiers de 1 à 5, dans l'art dentaire.

**6.** Utilisation suivant la revendication 5, caractérisée en ce que les esters d'acide (méth)acrylique de 1,2-bis(phénoxy)-3,3,4,4,5,5-hexafluoro-1-cyclopentènes substitués sont incorporés à des compositions d'obturation dentaire.

**7.** Utilisation suivant la revendication 5, caractérisée en ce que les esters d'acide (méth)acrylique de 1,2-bis(phénoxy)-3,3,4,4,5,5-hexafluoro-1-cyclopentènes substitués sont utilisés dans des compositions pour le revêtement de dents.

**8.** Utilisation suivant la revendication 5, caractérisée en ce que les esters d'acide (méth)acrylique de 1,2-bis(phénoxy)-3,3,4,4,5,5-hexafluoro-1-cyclopentènes substitués sont utilisés pour la production de dents en matière synthétique.

**9.** Matériaux dentaires durcissables, caractérisés en ce qu'ils contiennent des esters d'acide (méth)-acrylique de 1,2-bis(phénoxy)-3,3,4,4,5,5-hexafluoro-1-cyclopentènes substitués de formule

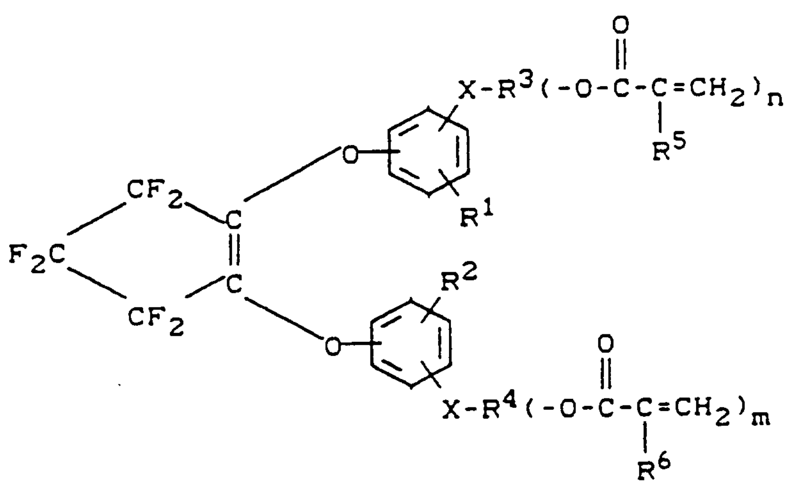

(I)

dans laquelle, indépendamment les uns des autres,

$R^1$, $R^2$ représentent un groupe alkyle en $C_1$ à $C_4$, un groupe halogénalkyle en $C_1$ à $C_4$ ou de l'hydrogène,

$R^3$, $R^4$ représentent des restes hydrocarbonés à chaîne droite ou ramifiés de valence (n + 1) ou (m + 1), ayant 2 à 15 atomes de carbone, qui peuvent contenir le cas échéant 1 à 3 ponts oxygène,

$R^5$, $R^6$ représentent de l'hydrogène ou un groupe méthyle et

X représente

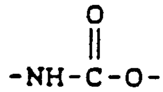

ou

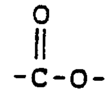

et

n et m sont des nombres entiers de 1 à 5.

**Revendications pour l'Etat contractant suivant : ES**

**1.** Procédé de production d'esters d'acide (méth)acrylique de 1,2-bis-(phénoxy)-3,3,4,4,5,5-hexafluoro-1-cyclopentènes substitués de formule (I)

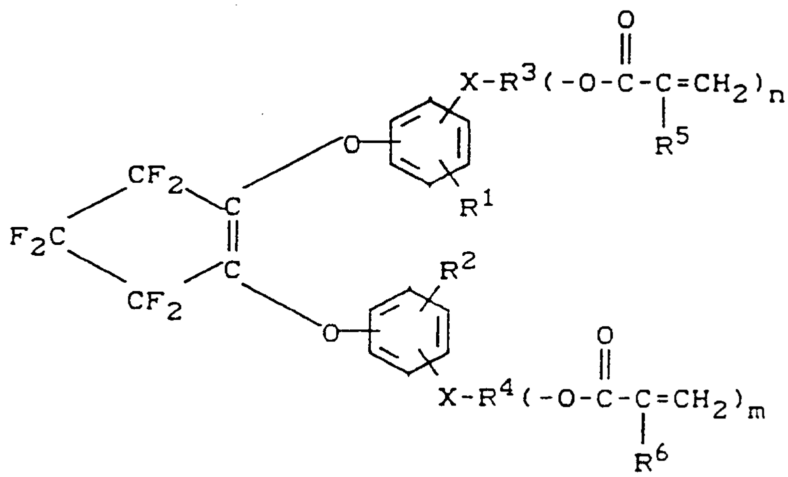

(I)

dans laquelle, indépendamment les uns des autres,

$R^1$, $R^2$ représentent un groupe alkyle en $C_1$ à $C_4$, un groupe halogénalkyle en $C_1$ à $C_4$ ou de l'hydrogène,

$R^3$, $R^4$ représentent des restes hydrocarbonés à chaîne droite ou ramifiée de valence (n + 1) ou (m + 1), ayant 2 à 15 atomes de carbone, qui peuvent contenir le cas échéant 1 à 3 ponts oxygène,

$R^5$, $R^6$ représentent de l'hydrogène ou un groupe méthyle et

X représente

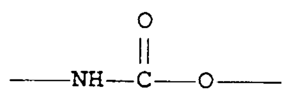 **ou** 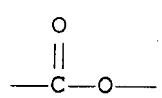

et

n et m sont des nombres entiers de 1 à 5.

caractérisé en ce qu'on fait réagir des 1,2-bis(phénoxy)-3,3,4,4,5,5-hexafluoro-1-cyclopentènes substitués de formule (II)

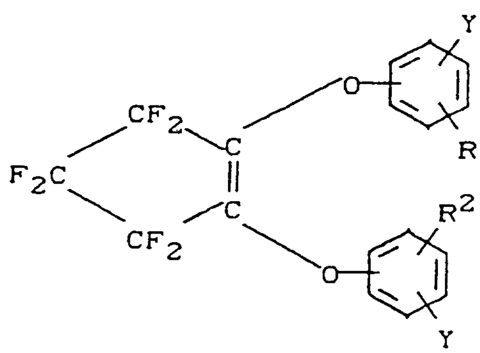

(II)

dans laquelle

$R^1$, $R^2$ représentent, indépendamment l'un de l'autre, un groupe alkyle en $C_1$ à $C_4$, un groupe

halogénalkyle en $C_1$ à $C_4$ ou de l'hydrogène et

Y est un groupe isocyanato (-NCO) ou chlorocarbonyle (-COCl),

avec au moins un dérivé d'acide (méth)acrylique à fonctionnalité OH, de formule

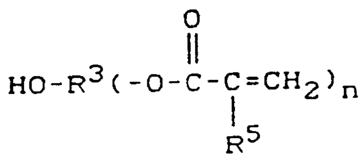

(III)

ou

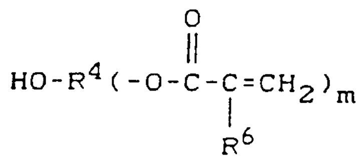

(IV)

dans laquelle, indépendamment les uns des autres,

$R^3$, $R^4$ sont des restes hydrocarbonés à chaîne droite ou ramifiés de valence (n + 1) ou (m + 1) ayant 2 à 15 atomes de carbone, qui peuvent contenir le cas échéant 1 à 3 ponts oxygène,

$R^5$, $R^6$ représentent de l'hydrogène ou un groupe méthyle et

n et m sont des nombres entiers de 1 à 5,

dans un solvant inerte, dans une plage de températures de -40 à +100 °C, le cas échéant en présence d'un catalyseur et/ou d'une base ainsi que, le cas échéant, en présence d'un inhibiteur de polymérisation.

**2.** Utilisation d'esters d'acide (méth)acrylique de 1,2-bis(phénoxy)-3,3,4,4,5,5-hexafluoro-1-cyclopentènes substitués de formule

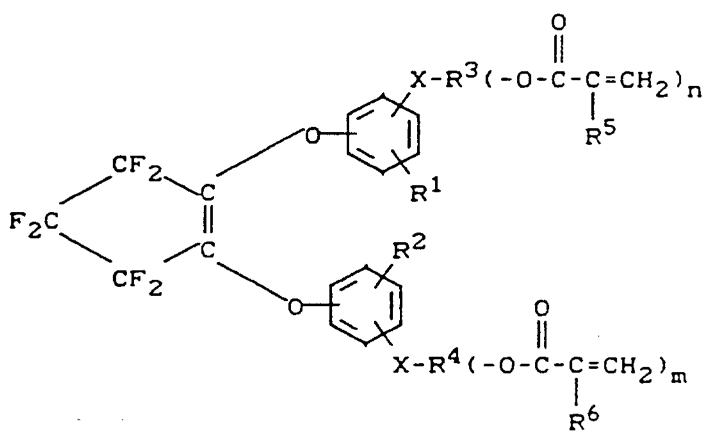

(I)

dans laquelle, indépendamment les uns des autres,

$R^1$, $R^2$ représentent un groupe alkyle en $C_1$ à $C_4$, un groupe halogénalkyle en $C_1$ à $C_4$ ou de l'hydrogène,

$R^3$, $R^4$ représentent des restes hydrocarbonés à chaîne droite ou ramifiés de valence (n + 1) ou (m + 1), ayant 2 à 15 atomes de carbone, qui peuvent contenir le cas échéant 1 à 3 ponts oxygène,

$R^5$, $R^6$ représentent de l'hydrogène ou un groupe méthyle et

X représente

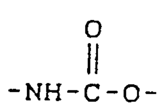

ou

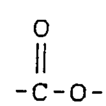

et

n et m sont des nombres entiers de 1 à 5, dans l'art dentaire.

3. Utilisation suivant la revendication 2, caractérisée en ce que les esters d'acide (méth)acrylique de 1,2-bis(phénoxy)-3,3,4,4,5,5-hexafluoro-1-cyclopentènes substitués sont utilisés dans des compositions d'obturation dentaire.

4. Utilisation suivant la revendication 2, caractérisée en ce que les esters d'acide (méth)acrylique de 1,2-bis(phénoxy)-3,3,4,4,5,5-hexafluoro-1-cyclopentènes substitués sont utilisés dans des compositions de revêtement de dents.

5. Utilisation suivant la revendication 2, caractérisée en ce que les esters d'acide (méth)acrylique de 1,2-bis(phénoxy)-3,3,4,4,5,5-hexafluoro-1-cyclopentènes substitués sont utilisés pour la fabrication de dents en matière synthétique.